(19) Europäisches Patentamt / European Patent Office / Office européen des brevets

(11) **EP 4 426 856 B1**

(12) **EUROPEAN PATENT SPECIFICATION**

(45) Date of publication and mention
of the grant of the patent:
**31.12.2025 Bulletin 2026/01**

(21) Application number: **22801526.9**

(22) Date of filing: **03.11.2022**

(51) International Patent Classification (IPC):
*C12Q 1/6827* (2018.01)

(52) Cooperative Patent Classification (CPC):
(C-Sets available)
**C12Q 1/6827** (Cont.)

(86) International application number:
**PCT/GB2022/052772**

(87) International publication number:
**WO 2023/079285 (11.05.2023 Gazette 2023/19)**

(54) **MULTIPURPOSE EDITING GENOTOXICITY ASSESSMENT (MEGA)**

MEHRZWECK-EDITIERUNGS-GENOTOXIZITÄTSBEURTEILUNG (MEGA)

ÉVALUATION DE GÉNOTOXICITÉ D'ÉDITION MULTIFONCTIONNELLE (MEGA)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR

(30) Priority: 03.11.2021 GB 202115834

(43) Date of publication of application:
**11.09.2024 Bulletin 2024/37**

(73) Proprietor: **UCL Business Ltd**
**London W1T 4TP (GB)**

(72) Inventors:
• **TURCHIANO, Giandomenico**
**London, Greater London W1T 4TP (GB)**
• **CAVAZZA, Alessia**
**London, Greater London W1T 4TP (GB)**
• **THRASHER, Adrian**
**London, Greater London W1T 4TP (GB)**
• **WHITE, Nathan**
**London, Greater London W1T 4TP (GB)**

(74) Representative: **Barker Brettell LLP**
**100 Hagley Road**
**Edgbaston**
**Birmingham B16 8QQ (GB)**

(56) References cited:
**WO-A1-2021/130271**

• BIO-RAD: "Droplet Digital ? PCR Droplet Digital ? PCR Applications Guide", 1 February 2010 (2010-02-01), XP055645030, Retrieved from the Internet <URL:http://www.bio-rad.com/webroot/web/pdf/lsr/literature/Bulletin_6407.pdf> [retrieved on 20191121]
• NAGY ERVIN D ET AL: "Novel disease resistance gene paralogs created by CRISPR/Cas9 in soy", PLANT CELL REPORTS, SPRINGER BERLIN HEIDELBERG, BERLIN/HEIDELBERG, vol. 40, no. 6, 11 March 2021 (2021-03-11), pages 1047 - 1058, XP037474704, ISSN: 0721-7714, [retrieved on 20210311], DOI: 10.1007/S00299-021-02678-5
• CURTIS B. HUGHESMAN ET AL: "A Robust Protocol for Using Multiplexed Droplet Digital PCR to Quantify Somatic Copy Number Alterations in Clinical Tissue Specimens", PLOS ONE, vol. 11, no. 8, 18 August 2016 (2016-08-18), pages e0161274, XP055590753, DOI: 10.1371/journal.pone.0161274
• A. DIDELOT ET AL: "Multiplex Picoliter-Droplet Digital PCR for Quantitative Assessment of DNA Integrity in Clinical Samples", CLINICAL CHEMISTRY, vol. 59, no. 5, 12 February 2013 (2013-02-12), pages 815 - 823, XP055197965, ISSN: 0009-9147, DOI: 10.1373/clinchem.2012.193409

EP 4 426 856 B1

- **PENG CHENG ET AL: "Accurate Detection and Evaluation of the Gene-Editing Frequency in Plants Using Droplet Digital PCR", FRONTIERS IN PLANT SCIENCE, vol. 11, 14 December 2020 (2020-12-14), CH, XP055931021, ISSN: 1664-462X, DOI: 10.3389/fpls.2020.610790**

(52) Cooperative Patent Classification (CPC): (Cont.)

C-Sets
**C12Q 1/6827, C12Q 2537/16, C12Q 2537/165, C12Q 2563/159, C12Q 2565/102**

**Description**

The present invention relates to a method for determining the level and type of mutation events associated with the use of a targeted genetic modification, such as in the use of designer nucleases/editors, to modify a target site of DNA in a cell population.

Targeted genetic modification, such as designer nuclease/editor technology, is becoming a standard procedure in many laboratories and it has revolutionized not only the basic biology research, but also the diagnostic and the gene therapy field leading to its application in several clinical trials.

Designer nuclease activity evaluation is routinely performed with PCR approaches that amplify a specific region surrounding the cleavage site within the window range of 300 to 700 bp. These methods can be used to evaluate presence of small insertions and deletions (indel) but fail to detect large deletions or mutations that disrupt one primer binding site. Other than the targeted sequence (On-target) editing evaluation, it is necessary to assess the safety of the designer nucleases to verify the quality and quantity of chromosomal aberrations induced at ON- and OFF- target sites by double strand breaks (DSBs). Off-target sites could be at any location in the chromosomal DNA, other than the intended target location, which may cause disruption of essential genes or regulatory sequences. WO2021130271 disclosed methods for determining the efficacy of genome editing methods by quantisation of target regions using digital PCR. Peng et al. (Frontiers in Plant Science, vol.11, 2020) developed a duplexed digital PCR-based method for the evaluation of gene-editing frequencies in plants.

Different techniques have been used to predict the quality and quantity of OFF-targets using *in-silico* (COSMID), *in-cellula (HTGTS, UDITAS, CAST-seq,* GUIDE-seq, IDLV integration, BLISS) or *in*-vitro (CIRCLE-seq, DIGENOME-seq) methodologies, but with poor resolution and/or accuracy. All these attributes are extremely relevant for gene therapy applications, where a standardised and fully unbiased technique aimed at evaluating the activity of designer nucleases is missing. Furthermore, even rates and quality of therapeutic transgene targeted integration may be over/underestimated or depending by specific cases, impossible to track and therefore surrogated to reporter gene expression constructs (GFP, Luciferase).

In order to tackle these requirements, it is desirable to develop techniques for a quick and unbiased overview of gene editing outcomes after targeted genetic modification, such as designer nuclease treatment, in therapeutically-relevant cells.

According to a first aspect of the present invention, there is provided a method for quantifying mutation events associated with a targeted genetic modification arranged to modify a target site of a targeted-chromosome in a cell population,

the method comprising carrying out a mutation event determination on a targeted chromosome of a modified cell population that has been treated with the targeted genetic modification, and a reference control analysis on a non-targeted chromosome,

wherein the reference control analysis comprises the use of digital PCR (dPCR), with first and second primer pairs designed to amplify respective first and second regions of the non-targeted chromosome in the modified cell population and in an unmodified cell population as a control,

wherein the dPCR further comprises a first labelled probe arranged to hybridise with and assay the level of the amplified first region of DNA and a second labelled probe arranged to hybridise with and assay the level of the amplified second region of DNA, wherein the labels of the first and second labelled probes are different to each other, wherein the relative quantity of dPCR droplets having combined first and second labelled probe detections relative to the quantity of dPCR droplets having first-only or second-only labelled probe detections is determined to quantify the level of genetic integrity of the non-targeted chromosome; and

wherein the mutation event determination on a modified cell population that has been treated with the targeted genetic modification comprises one or more analysis strategies selected from:

A) a flanking analysis to determine one or more mutation events including open ends, translocation and deletions, wherein the flanking analysis is conducted on the modified cell population and an unmodified cell population as a control,

the flanking dPCR analysis comprising the use of dPCR with a third primer pair to amplify a 5' region of DNA that is 5' to the target cleavage/editing site in the targeted chromosome and a fourth primer pair to amplify a 3' region of DNA that is 3' to the target cleavage/editing site,

wherein the dPCR further comprises a third labelled probe arranged to hybridise with the amplified 5' region of the targeted chromosome and a fourth labelled probe arranged to hybridise with the amplified 3' region of the targeted chromosome, wherein the labels of the third and fourth labelled probes are different to each other, wherein the relative level of amplified 5' and 3' regions of the targeted chromosome is determined by measuring the quantity of dPCR droplets having combined third and fourth (5' and 3') labelled probe detections relative to the quantity of dPCR droplets having third(5')-only or fourth(3')-only labelled probe detections to quantify the level of mutation events, and optionally, wherein the level of mutation events in the targeted chromosome is normalised against the level of genetic integrity of the non-targeted chromosome;

B) an on-target analysis to determine mutation events of aberrant insertions and/or deletions, wherein the on-target analysis is conducted on the modified cell population and an unmodified cell population as a control,

the on-target analysis comprising the use of dPCR with a fifth primer pair to amplify a region of DNA that includes the target cleavage/editing site in the targeted chromosome,
wherein the dPCR further comprises a fifth labelled probe arranged to hybridise with the target cleavage/editing site in the amplified DNA that has been modified by the targeted genetic modification and a sixth labelled probe arranged to hybridise with the amplified DNA at a site that is not the target cleavage/editing site, wherein the labels of the fifth and sixth labelled probes are different to each other,
wherein the level of mutation events associated with aberrant deletions and/or insertions at the target cleavage/editing site is determined by measuring the quantity of dPCR droplets having combined fifth and sixth (on-target and off-target) labelled probe detections indicating the presence of the expected genetic modification relative to the quantity of dPCR droplets having fifth(on-target)-only or sixth(off-target)-only labelled probe detections;

C) a loss of heterozygosity (LOH) analysis to determine the level of mutation events associated with aberrant chromosomal LOH in the targeted chromosome, wherein the LOH analysis is conducted on the modified cell population and an unmodified cell population as a control,

the LOH analysis comprising the use of dPCR with a sixth primer pair and a seventh primer pair to amplify respective 5' and 3' sub-telomeric regions of DNA at the extremities of the targeted chromosome,
wherein the dPCR further comprises a seventh labelled probe arranged to hybridise with and assay the level of amplified DNA of the 5' sub-telomeric region and an eighth labelled probe arranged to hybridise with and assay the level of amplified DNA of the 3' sub-telomeric region, wherein the labels of the seventh and eighth labelled probes are different to each other,
wherein the level of mutation events associated with LOH is determined by the copy number variation of either of the two LOH amplicons (5' and 3' sub-telomeric regions) in relation to the copy number of either of the amplicons as determined in the reference control analysis and the unmodified cell population control;

D) a knock-in and off-target integration (KI-OT) analysis to determine the level of events associated with integration of a donor DNA into the targeted chromosome and/or donor DNA present as episomal DNA, wherein the KI-OT analysis is conducted on the modified cell population and an unmodified cell population as a control,

the KI-OT analysis comprising the use of dPCR with an eighth primer pair to amplify a region of the donor DNA and a ninth primer pair to amplify a region of the genomic DNA of the targeted chromosome,
wherein the dPCR further comprises a ninth labelled probe arranged to hybridise with the amplified region of the donor DNA and a tenth labelled probe arranged to hybridise with the genomic region of DNA, wherein the labels of the ninth and tenth labelled probes are different to each other,
wherein the level of integration of the donor DNA into the targeted chromosome and/or donor DNA present as episomal DNA is determined by determining the quantity of dPCR droplets having combined ninth and tenth (donor and genomic) labelled probe detections, indicating linkage/integration, relative to the quantity of dPCR droplets having ninth(donor)-only or tenth(genomic)-only labelled probe detections.

The total level of mutagenesis events associated with a targeted genetic modification may be determined by combining the determined mutation events as determined by the one or more analysis strategies A, B, C and D.

Advantageously, the invention provides a method, herein named "MEGA" (Multipurpose Editing Genotoxicity Assessment), which provides a quick and unbiased overview of gene editing outcomes after targeted genetic modification, such as designer nuclease/editor treatment, in therapeutically-relevant cells. This methodology provides an overall single analysis that is more complete, rapid, higher-throughput and consistent than previously available to those assessing the potential genotoxicity of a targeted genetic modification agent or strategy. It takes advantage of digital PCR technology (dPCR) and enables the quantification of double strand breaks (DSBs) in the targeted sites, while discerning the large deletions and the chromosomal aberrations (such as translocations, inversions, unrepaired DSBs). It is also able to quantify the copy number variation of the entire targeted chromosome or the possible loss of 5' or 3' chromosome arm portions with respect to the cleavage site. In case of a gene addition approach, where a DNA donor template, either viral or oligonucleotide based, is utilized to knock-in genetic sequences at the ON-target site, this methodology can also detect and quantify the amount of integrated and episomal DNA fragment. Vectors sequences, such as lentiviral or AAV, can be investigated for their stability and integrity before and after transduction in the targeted cell population. This method can make use of few nanograms of genomic DNA (the gDNA amount can be scalable depending on the requested sensitivity) derived from therapeutically relevant primary cells and it complements standard and high-throughput techniques for indel quantification, off-target analysis and chromosomal aberration characterization and quantification.

**The targeted genetic modification**

The targeted genetic modification may derive from targeted nuclease or genome modifiers. The genetic modification may comprise the addition, deletion or substitution of one or more nucleotides in a nucleotide sequence. The genetic mutation may comprise a sequence insertion or deletion, or translocation. The genetic modification may comprise or consist of a single stranded cut (nick), or a double stranded break/cut (DSB). The double stranded cut may be blunt ended, or may leave overhangs, such as sticky ends.

The skilled person will recognise that a "targeted nuclease" may also be referred to as a "designer nuclease" and such terms may be used interchangeably throughout. Targeted nuclease modifications may comprise genetic modifications using the targeted nuclease.

The targeted nuclease may comprise or consist of a RNA-guided endonuclease (RGEN), such as CRISPR/-Cas9, Cas-CLOVER, mini-Cas9, or orthologues thereof. In another embodiment, the targeted nuclease may comprise or consist of zinc finger nuclease (ZFN) or transcription activator-like effector nuclease (TALEN).

In another embodiment, the targeted genetic modification may comprise or consist of base-editors, prime-editors or targeted transposons.

In another embodiment, the targeted genetic modification may be a viral vector integration into the DNA, such as genomic DNA.

In one embodiment the targeted nucleic acid is DNA. The targeted DNA may be genomic DNA. Alternatively, the targeted DNA may be mitochondrial DNA.

The targeted nucleic acid may comprise a target/recognition sequence of between 8 and 40 nucleotides. The skilled person will recognise that the length of target sequence required may depend on the targeted genetic modification technology used. For example, CRISPR/Cas9 may recognise a sequence of about 20 nucleotides.

In one embodiment, the targeted nucleic acid is part of a nucleic acid molecule/strand that acts as a template for the dPCR reaction.

The "target cleavage/editing site" may refer to the specific site of cleavage of the targeted genetic modification, for example a nick or double-stranded break for insertion, deletion or substitution of nucleotide residues.

The target cleavage/editing site may be in a gene or regulatory sequence. The target cleavage/editing site may be in gene region Xp11 or Xq22 for the WAS or BTK genes, respectively.

**The Analysis Strategies**

**Reference Control Analysis**

The two pairs of primers may be designed to obtain the same amplicon length, for example about 60-120 nucleotides in length. In a preferred embodiment, the first region of DNA may be between 60 and 120 nucleotides in length. In another embodiment, the first region of DNA may be between 40 and 2000 nucleotides in length.

In a preferred embodiment, the distance between the first and second regions of DNA to be amplified in the reference control assay may be about 150-250 nucleotides. In another embodiment, the distance between the first and second regions of DNA to be amplified in the reference control assay may be about 0-10 kb in length.

In a preferred embodiment, the second region of DNA may be between 60 and 120 nucleotides in length. In another embodiment, the second region of DNA may be between 40 and 2000 nucleotides in length.

In one embodiment, the reference control analysis is used to quantify the genomic or DNA integrity. The target first and second regions of this analysis strategy may be located on a chromosome different to that of the chromosome targeted for the targeted genetic modification, such as nuclease cleavage. This may avoid variations in the reference control analysis strategy, such as from potential chromosomal aberrations that may occur in the targeted chromosome, i.e., LOH.

The relative level (e.g., ratio) of amplified first and second regions of DNA in the non-target chromosome may be determined by measuring the quantity of dPCR droplets having combined first and second labelled probe detections relative to the quantity of dPCR droplets having first-only or second-only labelled probe detections. The genomic integrity/fragmentation may be calculated by the ratios among the single positive droplets with the double positive droplets.

The values obtained from the non-targeted chromosome in the modified cell population may be normalised against the values obtained in the unmodified cell population as a control.

**Flanking Analysis**

The two pairs of primers may be designed to obtain the same amplicon length, for example about 60-120 nucleotides in length. In a preferred embodiment, the 5' region of DNA that is 5' to the target cleavage/editing site in the targeted chromosome may be between 60 and 120 nucleotides in length. In another embodiment, the 5' region of DNA that is 5' to the target cleavage/editing site in the targeted chromosome may be between 40 and 2000 nucleotides in length.

The distance between the 5' and 3' regions of DNA to be amplified in the flanking analysis may be at least 40 nucleotides. In a preferred embodiment, the distance between the 5' and 3' regions of DNA to be amplified in the flanking analysis may be about 40-400 nucleotides. In another embodiment, the distance between the 5' and 3' regions of DNA to be amplified in the flanking analysis may be about 40-200 nucleotides. In another embodiment, the distance between the 5' and 3' regions of DNA to be amplified in the flanking analysis may be about 0-10kb.

The 5' region of DNA that is 5' to the target cleavage/editing site in the targeted chromosome may be at least 20 nucleotides from the target cleavage/editing site. The 5' region of DNA that is 5' to the target cleavage/editing site in the targeted chromosome may be within 200 nucleotides of the target cleavage/editing site. In another embodiment, the 5' region of DNA that is 5' to the target cleavage/editing site in the targeted chromosome may be distanced between 20 and 200 nucleotides from the target cleavage/editing site. In another embodiment, the 5' region of DNA that is 5' to the target cleavage/editing site in the targeted chromosome may be distanced 0-10kb from the target cleavage/editing site.

In a preferred embodiment, the 3' region of DNA that is 3' to the target cleavage/editing site in the targeted chromosome may be between 60 and 120 nucleotides in length. In another embodiment, the 3' region of DNA that is 3' to the target cleavage/editing site in the targeted chromosome may be between 40 and 2000 nucleotides in length.

The 3' region of DNA that is 3' to the target cleavage/editing site in the targeted chromosome may be at least 20 nucleotides from the target cleavage/editing site. The 3' region of DNA that is 3' to the target cleavage/editing site in the targeted chromosome may be within 200 nucleotides of the target cleavage/editing site. In another embodiment, the 3' region of DNA that is 3' to the target cleavage/editing site in the targeted chromosome may be distanced between 20 and 200 nucleotides from the target cleavage/editing site. In another embodiment, the 3' region of DNA that is 3' to the target cleavage/editing site in the targeted chromosome may be distanced 0-10kb from the target cleavage/editing site.

The relative level of amplified 5' and 3' regions of DNA in the targeted chromosome may be determined by measuring the quantity of dPCR droplets having combined third and fourth (5' and 3') labelled probe detections relative to the quantity of dPCR droplets having third(5')-only or fourth(3')-only labelled probe detections.

The linkage percent (i.e. between the probed sequences, which is represented by the double positive droplets) may be normalized by the genomic integrity derived in the reference control analysis and may represent gross chromosomal aberrations. The copy number variation between the flanking analysis and the reference control analysis may represent large deletions.

The values obtained from the non-targeted chromosome in the modified cell population may be normalised against the values obtained in the unmodified cell population as a control.

In one embodiment, the values in the targeted chromosome is normalised against the values in the reference control of the non-targeted chromosome, and normalised against the relative values obtained from the unmodified cell population control.

The same approach can be multiplexed with assays, carrying different dyes, designed over the vector of interest to study its integrity before and after transduction.

**On-target analysis**

In one embodiment, the fifth primer pair to amplify a region of DNA that includes the target cleavage/editing site in the targeted chromosome, may comprise a forward primer that is 5' to the target cleavage/editing site and a reverse primer that is 3' to the target cleavage/editing site.

The "fifth labelled probe" may otherwise be termed an "on-target probe". The fifth labelled probe arranged to hybridise with the point of cleavage at the target in the amplified DNA that has been modified by the targeted genetic modification may be arranged to span the cleavage/edited site. It is understood that the sequence of the target cleavage/editing site of the modified nucleic acid will be the desired/designed sequence after the successful modification by the targeted genetic modification. The fifth labelled probe may be capable of spanning the entire genetic modification region. Alternatively, the fifth labelled probe may span between an unmodified/non-targeted region of the nucleic acid and a modified region comprising the genetic modification, for example in cases where the genetic modification is an insertion that is longer than the probe. The fifth labelled probe may hybridise to a region that spans between juxtaposed sequences of the original/unmodified sequence and an insert sequence, or juxtaposed sequences of the original/unmodified sequence having a deletion therebetween. The juxtaposed sequences at the target cleavage/editing site following genetic modification, or nucleotide substitutions at the target cleavage/editing site, may form a new sequence that would be unique for targeting by the probe.

In one embodiment, the fifth labelled probe may comprise a minor groove binding domain (MGB), for example to increase the sensitivity towards mutations.

The "sixth labelled probe" may otherwise be termed a "distal-target probe". In a preferred embodiment, the sixth labelled probe arranged to hybridise with the amplified DNA at a site that is not the target cleavage/editing site may be targeted at a region that is at least 20 nucleotides far from the cleavage site. In another embodiment, the sixth labelled probe arranged to hybridise with the amplified DNA at a site that is not the target cleavage/editing site may be targeted at a region that is 0-10kb far from the cleavage site.

The relative level of on-target versus distal-target probe hybridisations in the targeted chromosome may be determined by measuring the quantity of dPCR droplets having combined fifth and sixth (on-target and distal-target) labelled probe detections (indicating the presence of unmodified sequence) relative to the quantity of dPCR droplets having sixth(distal-target)-only labelled probe detections (indicating the presence of genetic modification or unknown genetic modification).

The values obtained from the non-targeted chromosome in the modified cell population may be normalised against the values obtained in the unmodified cell population as a control. The double positive (two probe) copy number difference from the reference control analysis and the unmodified control sample may be used to determine the absolute amount of small indels, large deletions and chromosomal aberrations together, including a targeted integration from a desired genetic modification.

### The loss of heterozygosity (LOH) analysis

In a preferred embodiment, the targeted sub-telomeric regions arranged to be amplified by the sixth primer pair and a seventh primer pair may be within 20 megabases of the respective telomere end. In another embodiment, the targeted sub-telomeric regions arranged to be amplified by the sixth primer pair and a seventh primer pair may be within 0-100Mb of the respective telomere end. It is preferable to design the amplification regions on unique sequence regions as far as possible from the target cleavage/editing site to ensure that all potential mutations detected within the targeted chromosome are LoH.

The amplified regions of sub-telomeric regions arranged to be amplified by the sixth primer pair and a seventh primer pair may be preferentially between about 60 and 120 nucleotides in length.

The copy number variation of one of the two 5' and 3' sub-telomeric amplicons and the reference control assay estimates the loss of heterozygosity in the targeted chromosome.

### The knock-in and off-target integration (KI-OT) analysis

The eighth primer pair is designed to recognise specifically the donor DNA sequence.

The ninth primer pair to amplify a region of the genomic DNA, may amplify a region that is close to the target cleavage/editing site. In a preferred embodiment, the ninth primer pair to amplify a region of the genomic DNA, may amplify a region that is outside a homology region utilized in the donor DNA and/or at least 50 bp from the cleavage site. In one embodiment, the ninth primer pair to amplify a region of the genomic DNA, may amplify a region that is outside a homology region utilized in the donor DNA and/or 0-10 kb from the cleavage site.

The relative level of amplified donor and genomic regions of DNA in the modified cell population may be determined by measuring the quantity of dPCR droplets having combined ninth and tenth (donor and genomic) labelled probe detections (indicating they are linked and there has been a donor integration) relative to the quantity of dPCR droplets having ninth(donor)-only or tenth(3' genomic)-only labelled probe detections (indicating no linkage and no site specific integration - the donor remains episomal).

The linkage percent (i.e., where the linked probed sequences are represented by double positive droplets) may represent the amount of targeted integration. Single positive droplets derived by the donor specific probe may represent the amount of donor DNA integrated in the genomic DNA and/or in an episomal state. The linkage percent (i.e., represented by double positive droplets) may be normalized by the genomic integrity derived in the reference control assay. Additionally, or alternatively, the copy number ratio determined between the KI-OT assay and the reference control assay may represent the amount of donor DNA not integrated in the targeted locus. In one embodiment, the values obtained from the non-targeted chromosome in the modified cell population may be normalised against the values obtained in the unmodified cell population as a control.

**Knock-in analysis (in-out strategy)**

In one embodiment, a further analysis is provided as follows:

E) a knock-in analysis to determine the level of events associated with integration of a donor DNA into targeted genomic DNA of the targeted chromosome,
the knock-in analysis comprising the use of dPCR with an eleventh primer pair to amplify a region of DNA comprising genomic and donor DNA, wherein the amplified region spans the join between the genomic DNA and the donor DNA, wherein the dPCR further comprises an eleventh labelled probe arranged to hybridise with the amplified region of genomic and donor DNA,
wherein the level of the amplified region of the genomic DNA and donor DNA in the targeted chromosome of the modified cell population indicates the level of integration of the donor DNA into the genomic DNA.

The knock-in analysis may be conducted on the modified cell population. The copy number ratio may be determined against a control amplicon of the same amplicon size. The control amplicon may be distanced away from the cleavage site or may be on a non-targeted chromosome.

**Assay Combinations**

Combinations of the analysis strategies on the treated cell population may be conducted in parallel or conducted sequentially. Two or more, or all, of the analysis strategies on the treated cell population may be carried out in separate dPCR reactions (e.g., may not share reagents). In another embodiment, two or more analysis strategies may be provided in the same dPCR reaction (e.g., may share reagents). Where combinations of analysis strategies are conducted in the same dPCR reaction, the labelled probes may be distinguishable between the different analysis strategies, for example by fluorescing at different wavelengths.

Where two or more, or all the analysis strategies are conducted, the nucleic acid from the modified cell population may be from the same population/culture and/or the same targeted genetic modification. In particular, portions of the modified nucleic acid may be distributed into two or more analysis strategies to be run in parallel or sequentially.

**The primer pairs**

The primers described herein may be any suitable length for priming a dPCR reaction. In one embodiment, the primers are at least 8 nucleotides in length. In another embodiment, the primers are about 8-40 nucleotides in length. In another embodiment, the primers are about 10-40 nucleotides in length. In another embodiment, the primers are about 8-30 nucleotides in length. In another embodiment, the primers are about 10-30 nucleotides in length. In another embodiment, the primers are about 15-25 nucleotides in length.

Where combinations of analysis strategies are conducted in the same dPCR reaction, the primer pairs may have substantially similar or compatible melting and annealing temperatures, for example the melting and annealing temperatures (Tm) of a primer pair for each analysis strategy may be within 5°C or preferably within 3°C of the respective melting and annealing temperatures of the primer pair of another analysis strategy.

The primers may comprise oligonucleotide, such as DNA, or nucleotide analogues thereof. Nucleotide analogues may comprise LNA (locked nucleic acid), PNA (peptide nucleic acid), PMO (phosphorodiamidate morpholino oligomer) or combinations thereof.

The primer pairs may be selected from any of the primer pairs provided in Tables 1-5 herein, or combinations thereof. The skilled person may select appropriate primer pairs and combinations in accordance with the analysis strategies being conducted.

**The Labelled Probes**

In one embodiment, the label of the labelled probes is a fluorescent label. The labelled probes may be 5' labelled with a fluorescent dye, such as a fluorescein. In one embodiment, the labelled probes may be labelled with fluorescein, such as fluorescein amidite (FAM) or 2'-chloro-7'phenyl-1,4-dichloro-6-carboxy-fluorescein (VIC). The fluorescein amidite may be 6-FAM.

The skilled person will recognise that any suitable fluorescent dyes may be used to label the probes herein, for example labelled probes may be selected from fluorescein amidite (FAM), TET, 2'-chloro-7'phenyl-1,4-dichloro-6-carboxy-fluorescein (VIC), hexachloro-fluorescein (HEX), and Cy3.5, or fluorescent dyes providing substantially similar wavelength emissions.

Where a pair of probes is used in an analysis strategy, the probes may be labelled differently, such that they are distinguishable. For example, a pair of probes in an analysis strategy may comprise two sets of oligonucleotides, where the two sets are labelled with different fluorescent dyes. In an embodiment where combinations of probes and/or analysis strategies are provided in a single dPCR reaction, each probe type may be labelled differently, such that they are distinguishable according to their excitation wavelength.

The labelled probes described herein may be any suitable length for specifically hybridising to a substantially complementary target sequence. In one embodiment, the labelled probes are at least 6, 7, 8, 9 or 10 nucleotides in length. In another embodiment, the labelled probes are about 8-40 nucleotides in length. In another embodiment, the labelled probes are about 10-40 nucleotides in length. In another embodiment, the labelled probes are about 8-30 nucleotides in length. In another embodiment, the labelled probes are about 10-30 nucleotides in length. In another embodiment, the labelled probes are about 15-25 nucleotides in length.

Where two or more labelled probes are used in an assay, the two or more labelled probes may have substantially similar or compatible annealing temperatures to their respective target sequences, for example the annealing temperatures may be within 10°C or preferably within 3°C of each other. In another embodiment, the annealing temperatures may be within 10% or preferably within 5% of each other.

Where combinations of analysis strategies are conducted in the same dPCR reaction, the labelled probes may have substantially similar or compatible annealing temperatures to their respective target sequences, for example the annealing temperatures of labelled probes for each assay may be within 10% or preferably within 5% of the respective annealing temperatures of the labelled probes of another analysis strategy. In another embodiment, the annealing temperatures may be within 10°C or preferably within 3°C of each other.

The Tm of the labelled probes may be about 5-15 °C higher than the primers. The labelled probes may further comprise a Minor Groove Binding (MGB) domain, for example to increase the annealing temperature and improve the positive signal intensity over the background and the sensitivity towards mutations.

The labelled probes may comprise oligonucleotide, such as DNA, or nucleotide analogues thereof. Nucleotide analogues may comprise LNA, PNA, PMO or combinations thereof.

The labelled probes may be selected from any of the labelled probes provided in Tables 1-5 herein, or combinations thereof. The skilled person may select appropriate probe combinations in accordance with the analysis strategy being conducted.

### The dPCR labelled probe detections in droplets

dPCR droplets may be individually measured and quantified for labelled probe detections, for example by a dPCR droplet reader, which scans each droplet for the labels of successfully hybridised probes, such as scanning for fluorescent wavelengths emitted by the probe labels. dPCR droplets may be sorted and counted using an adapted FACS method to sort and count droplets instead of cells, for example with a (FADS fluorescent activated droplet sorter) device specific for the droplets.

### The cell population

The cell population may comprise cells that are prokaryotic or eukaryotic. Preferably the cells are eukaryote cells. In one embodiment the cells are mammalian cells, such as human cells.

The cells may be stem cells, such as iPSCs or ESCs. The cells may be germline or somatic cells. In one embodiment the cells may be immune cells, such as lymphocytes (T cells, B cells or natural killer (NK) cells), neutrophils, and monocytes/macrophages. The cells may comprise a mixed population of cell types.

In one embodiment, the cells may be associated with a disease or condition, such as cancer cells or infected cells. In one embodiment, the cells may be associated with a mutation or infection causing a disease or condition.

The modified cell population and unmodified cell population may be of the same cell type and/or source. In particular, the modified cell population and unmodified cell population may be substantially identical, other than the modified cell population has been treated with a targeted genetic modification.

The cell number may be sufficient for a treatment. In one embodiment, at least 1000 cells are provided. Preferably at least 50,000 cells are provided.

### DNA extraction method

The DNA of the unmodified- and modified cell population may be extracted from the cells such that it is suitable as a template for the dPCR. Preferably, the genomic DNA is extracted from the cells of the unmodified- and modified cell population.

The skilled person will be familiar with various genomic DNA extraction techniques that may be used. In a preferred embodiment, the genomic DNA is extracted with a technique that maintains a high degree of genomic integrity with an average fragment length size >15 kb. In one embodiment, the genomic DNA is extracted by the salting-out method, for example as described by Miller et al (Nucleic Acids Research, 1988, 16, 1215). Suitable high molecular weight extraction methods may be used by the skilled person, for example by glass-bead precipitation, such as provided by the Monarch® HMW DNA Extraction Kit (New England Biolabs Inc.).

DNA extraction methods can damage the DNA creating fragments of all sizes. The method of the invention advantageously takes the genomic integrity into account and can further use an extraction technique providing a more intact genome, thereby increasing the accuracy of the determination.

**The dPCR**

In one embodiment, extracted DNA from the cell population may be digested into smaller fragments, for example by restriction enzyme digestion. This may facilitate distribution of the DNA into the droplets of the dPCR. Preferably, the restriction enzyme cleavage sites are not located in or close (e.g. 2bp or less) to the assays of interest to prevent interference of the amplification and analysis.

Distribution of the nucleic acid may be facilitated by dilution of the nucleic acid. Therefore, in one embodiment, the nucleic acid solution may be diluted to be in the correct range of quantification. For example, Poisson distribution may be used for the absolute quantification calculation.

The amplification reagent for dPCR DNA amplification may otherwise be termed a "master mix" or "amplification mix". The skilled person will understand that an amplification mix may comprise all the reagents necessary for droplet generation and PCR amplification of the DNA. Such components may comprise reaction buffer, polymerase, and dNTPs. A DNA polymerisation reporter molecule, such as a DNA-binding dye (e.g., EvagreenTM) may also be provided in the amplification mix, for example to allow monitoring of the amplification reaction using a real-time PCR. The DNA-binding dye may be constructed of two monomeric DNA-binding dyes linked by a flexible spacer. In the absence of DNA, the dimeric dye can assume a looped conformation that is inactive in DNA binding. When DNA is available, the looped conformation can shift via an equilibrium to a random conformation that is capable of binding to DNA to emit fluorescence.

The amplification reagents may be divided equally between the dPCR droplets.

The skilled person will be able to provide suitable conditions for the amplification reaction to occur, including suitable temperature and incubation times.

About 20-30ng of the nucleic acid, such as human gDNA, may be provided for distribution within the droplets. In another embodiment, 10-100ng of nucleic acid, such as gDNA, may be provided for distribution within the droplets (e.g.,, for diploid human genomic DNA). In another embodiment, 25-100ng of nucleic acid, such as gDNA, may be provided for distribution within the droplets.

Alternatively, about 10-20ng of the nucleic acid, such as human gDNA, may be provided for distribution within the droplets. In another embodiment, 5-50ng of nucleic acid, such as gDNA, may be provided for distribution within the droplets (e.g.,, for diploid human genomic DNA).

The amount of DNA may be sufficient to result in no more than 20% of positive droplets for the analysis strategy of interest. This advantageously avoids formation of double positive droplets by chance and can reduce the impact of the normalization for genomic integrity.

The final concentration of primers may be about 1 $\mu$M. In one embodiment, the final concentration of primers may be about 0.2-1 $\mu$M.

The final concentration of labelled probes may be about 250 nM. In one embodiment, the final concentration of labelled probes may be about 50-500 nM.

The dPCR may be conducted with at least 1000 droplets. Preferably at least 10000 droplets per analysis is used. The droplets' size and volume may be consistent/standardised (i.e., substantially equal) within the population of droplets.

The dPCR droplet preparation, reaction and processing may be conducted by a suitable dPCR system and a reader, such as the QX200™ Droplet Reader/QuantaSoft™ Analysis Pro Software (Bio-Rad Laboratories), naica® system - Multiplex Crystal Digital PCR™/Crystal Miner Software (STILLA technologies), or QIAcuity Digital PCR System/ QIAcuity Software Suite.

According to another aspect of the present invention, there is provided the use of the method of the invention herein for screening potential targeted genetic modification agents, such as designer nucleases, for therapeutic use.

The skilled person will recognise that the method of the invention may be adapted to determine the genomic or DNA integrity of any nucleic acid, such as a vector, or a viral genomic nucleic acid (e.g., viral DNA).

The targeted genetic modification may be in any nucleic acid type. Therefore, the term "targeted-chromosome in a cell population" may be substituted with "targeted nucleic acid in a nucleic acid population". For example, the "cell population" may be substituted herein with a "virus population", and the "targeted-chromosome" and "non-targeted chromosome" may be a "targeted viral genome" and non-targeted viral genome" respectively. In a further example, the "cell population" may be substituted herein with a "microbial population", and the "targeted-chromosome" and "non-targeted chromosome" may be a "targeted genome" and non-targeted genome" respectively.

According to another aspect of the present invention, there is provided a method for quantifying mutation events associated with a targeted genetic modification arranged to modify a target site of a nucleic acid, such as DNA or RNA,

the method comprising carrying out a mutation event determination on a targeted nucleic acid in a population of modified nucleic acids that have been treated with the targeted genetic modification, and a reference control analysis on a non-targeted nucleic acid,

wherein the reference control analysis comprises the use of digital droplet PCR (dPCR) with first and second primer pairs designed to amplify respective first and second regions of the non-targeted nucleic acid and in an unmodified nucleic acid as a control,

wherein the dPCR further comprises a first labelled probe arranged to hybridise with and assay the level of the amplified first region of DNA and a second labelled probe arranged to hybridise with and assay the level of the amplified second region of DNA, wherein the labels of the first and second labelled probes are different to each other, wherein the relative quantity of dPCR droplets having combined first and second labelled probe detections relative to the quantity of dPCR droplets having first-only or second-only labelled probe detections is determined to quantify the level of genetic integrity of the non-targeted nucleic acid; and

wherein the mutation event determination on a modified nucleic acid that has been treated with the targeted genetic modification comprises one or more analysis strategies selected from:

1) a flanking analysis to determine one or more mutation events including open ends, translocations, and deletions, wherein the flanking analysis is conducted on the modified nucleic acid population and an unmodified nucleic acid population as a control,

the flanking dPCR analysis comprising the use of dPCR with a third primer pair to amplify a 5' region of DNA that is 5' to the target cleavage/editing site in the targeted nucleic acid and a fourth primer pair to amplify a 3' region of DNA that is 3' to the target cleavage/editing site,

wherein the dPCR further comprises a third labelled probe arranged to hybridise with the amplified 5' region of the targeted nucleic acid and a fourth labelled probe arranged to hybridise with the amplified 3' region of the targeted nucleic acid, wherein the labels of the third and fourth labelled probes are different to each other, wherein the relative level of amplified 5' and 3' regions of the targeted nucleic acid is determined by measuring the quantity of dPCR droplets having combined third and fourth (5' and 3') labelled probe detections relative to the quantity of dPCR droplets having third(5')-only or fourth(3')-only labelled probe detections to quantify the level of mutation events, and optionally, wherein the level of mutation events in the targeted nucleic acid is normalised against the level of genetic integrity of the non-targeted nucleic acid;

2) an on-target analysis to determine mutation events of aberrant insertions and/or deletions, wherein the on-target analysis is conducted on the modified nucleic acid population and an unmodified nucleic acid population as a control,

the on-target analysis comprising the use of dPCR with a fifth primer pair to amplify a region of DNA that includes the target cleavage/editing site in the targeted nucleic acid,

wherein the dPCR further comprises a fifth labelled probe arranged to hybridise with the target cleavage/editing site in the amplified DNA that has been modified by the targeted genetic modification and a sixth labelled probe arranged to hybridise with the amplified DNA at a site that is not the target cleavage/editing site, wherein the labels of the fifth and sixth labelled probes are different to each other, wherein the level of mutation events associated with aberrant deletions and/or insertions at the target cleavage/editing site is determined by measuring the quantity of dPCR droplets having combined fifth and sixth (on-target and off-target) labelled probe detections indicating the presence of the expected genetic modification relative to the quantity of dPCR droplets having fifth(on-target)-only or sixth(off-target)-only labelled probe detections;

3) a knock-in and off-target integration (KI-OT) analysis to determine the level of events associated with integration of a donor DNA into the targeted nucleic acid and/or donor DNA present as separate DNA, wherein the KI-OT analysis is conducted on the modified nucleic acid population and an unmodified nucleic acid population as a control,

the KI-OT analysis comprising the use of dPCR with a primer pair to amplify a region of the donor DNA and a primer pair to amplify a region of the genomic DNA of the targeted nucleic acid,

wherein the dPCR further comprises a labelled probe arranged to hybridise with the amplified region of the donor DNA and a labelled probe arranged to hybridise with the targeted nucleic acid, wherein the labels of the two labelled probes are different to each other,

wherein the level of integration of the donor DNA into the targeted nucleic acid and/or donor DNA present as separate DNA is determined by determining the quantity of dPCR droplets having combined donor and targeted nucleic acid labelled probe detections, indicating linkage/integration, relative to the quantity of dPCR droplets having donor-only or targeted nucleic acid-only labelled probe detections.

The total level of mutagenic events associated with a targeted nucleic acid modification may be determined by combining the determined mutation events as determined by the one or more analysis strategies 1, 2, and 3.

The nucleic acid may be DNA, such as vector DNA. In another embodiment, the nucleic acid may be a viral genome, such as viral genomic DNA. In another embodiment the nucleic acid may be bacterial DNA.

**Definitions**

The term "digital PCR (dPCR)" refers to a method for performing digital PCR that is based on water-oil emulsion droplet or physical partitioning technology. A sample of nucleic acid with a master mix of reagents is spread into physical partitions or fractionated into thousands of droplets (e.g., 20,000 droplets) with the aim of the droplets/partitions only having a single targeted nucleic acid molecule. A simultaneous PCR amplification reaction is carried out on the targeted template nucleic acid molecules present in the individual droplets. The term "digital PCR (dPCR) may be used interchangeably with "digital droplet PCR (ddPCR)".

Genotoxicity describes the property of an agent able to alter the genetic function within a cell causing unwanted mutations/effects, which may lead to functional impairment or disease development (e.g., cancer, therapy impairment, differentiation impairment).

Reference herein to an "insertion" is understood to mean a genetic modification that involves a sequence of nucleic acid being inserted into the sequence of another nucleic acid.

Reference herein to a "deletion" is understood to mean a genetic modification that involves a sequence of nucleic acid being removed, or otherwise termed "deleted".

Reference herein to an "indel" is understood to mean a genetic modification that may be an insertion or deletion.

"Translocation" refers to a type of chromosomal abnormality in which a chromosome and a portion of it recombines to a different chromosome.

"Loss of heterozygosity (LOH)" is understood to be a gross chromosomal event that results in loss of chromosomal regions.

"Homologous recombination" is understood to be a type of genetic recombination in which genetic information is exchanged between two similar or identical nucleic acid molecules.

Reference herein to "donor DNA" or "DNA donor" is understood to mean a sequence of DNA that has been provided from a non-chromosomal sequence, such as synthetic DNA, or a vector.

The skilled person will understand that optional features of one embodiment or aspect of the invention may be applicable, where appropriate, to other embodiments or aspects of the invention.

Embodiments of the invention will now be described in more detail, by way of example only, with reference to the accompanying drawings.

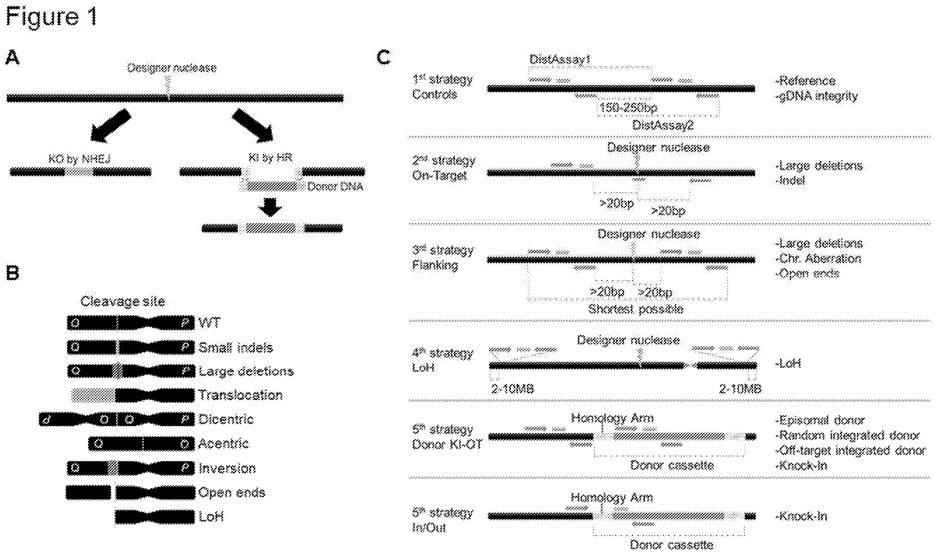

**Figure 1. Experimental design**

A) Designer nuclease activity schematic are utilized to induce Non-Homologous End Joining (NHEJ) or Knock-In of a Donor DNA sequence in the cleaved site. B) DSB possible outcomes after designer nuclease editing causing different chromosomal aberrations that can be measured by MEGA analysis. C) Schematic description of the different dPCR assays deployed for the MEGA. The 5th assay is displayed in two configurations: the "KI-OT" measuring donor copies inside and outside the targeted locus and the "In/Out" measuring the donor copies integrated in the Locus.

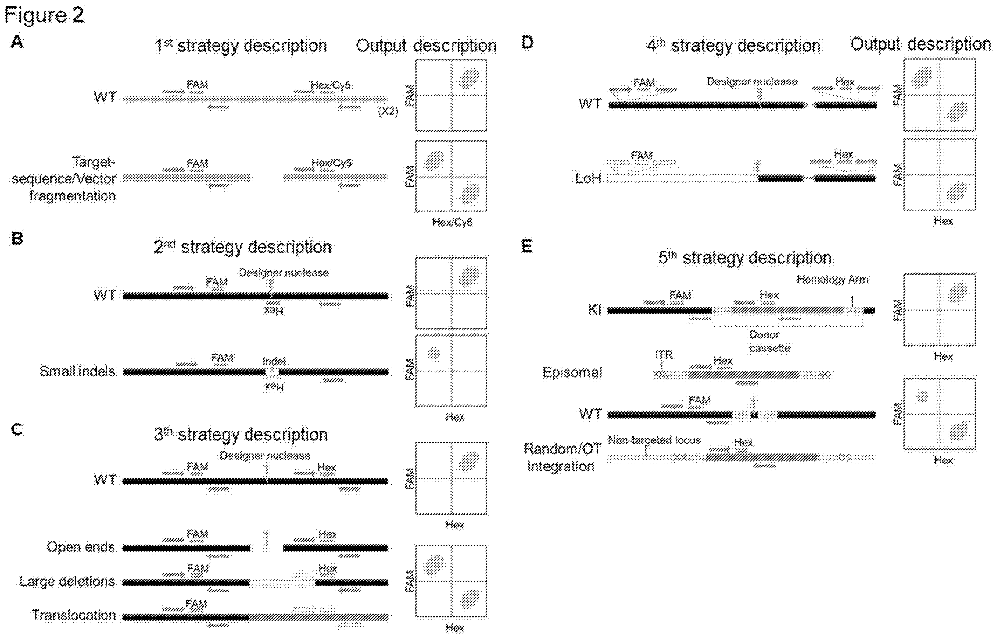

**Figure 2. Assays outputs description**

A) 1st assay output to test the sample genomic integrity and its quantity. These assays are designed in a non-targeted chromosome to serve as reference for the subsequent assays. The two coupled assays designed at a known distance would also measure the genomic integrity/fragmentation calculated by the ratios among the single positive droplets with the double positives. A second set of coupled primers located on another editing independent locus can also be used as a refence and the output averaged to reduce variability when normalising the subsequent assays.

B) 2nd assay output description to test the amount of small indels present in the immediate surroundings of the cleavage site. Double positive droplets are expected when the sequence is not mutated close to the cleavage site.

Single positive droplets will be instead counted when mutations will perturb the probe designed over the cleavage site. The two-probe copy number difference will determine the absolute amount of small indels. The difference between the quantity of copies retrieved by the double positive droplets and the reference (1st assay) would also indicate chromosomal aberrations including the targeted integration and large deletions.

C) 3rd "flanking" assay schematic output. The linkage percentage calculated by the two coupled primers and probes designed in proximity of the cleavage site will represent the amount of chromosomal aberrations such as large deletions, translocations, or open DNA ends at cleavage site. The linkage percentage can be normalized by the sample genomic integrity derived in the 1st assay. In addition, the copy number variation between the assays in C and the reference assay in A will represent the large deletions.

D) 4th assay schematic output for the loss of heterozygosity calculation. Copy number variation among one of the two assays in D and the reference in A will estimate the loss of heterozygosity in the targeted chromosome.

E) 5th KI-OT assay schematic output. The linkage percentage calculated by the two couple d primers and probes designed outside the homology arm and specifically for the donor DNA template will represent the amount of targeted integration. Single positive signal derived by the donor specific assay represent the amount of donor DNA integrated in the gDNA and/or in an episomal state. The linkage percentage can be normalized by the sample genomic integrity derived in the 1st assay. In addition, the copy number ratio between the donor specific assay in E and the reference assay in A will represent the amount of donor DNA not integrated in the targeted locus.

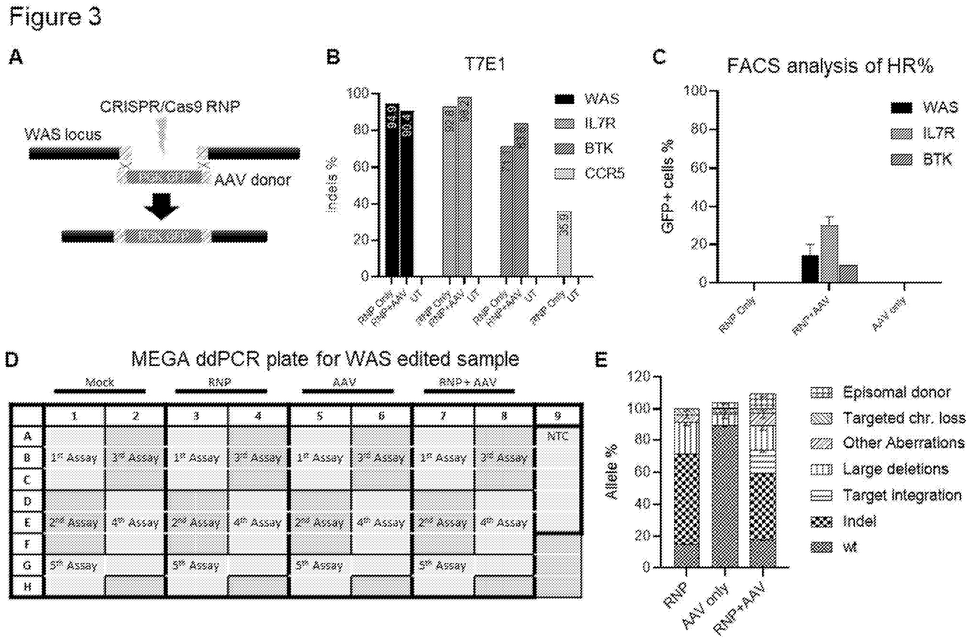

**Figure 3. Gene editing of therapeutic targets**

A) AAV donor DNA sequence harbouring PGK-GFP expression cassette cloned between homology arms for the relative targeted sequences (WAS, BTK, IL7R, CCR5). CRISPR/Cas9 RNPs are electroporated to induce DSB with and without the AAV donor transduction. B) T7 endonuclease1 (T7E1) assay was performed to estimate the CRISPR/Cas9 cleavage activity in the targeted sites. The AAV only and untreated (UT) samples were also analysed as negative control. C) Treated cells were analysed via flow cytometry to estimate the homologous recombination rate. D) Schematic of a MEGA panel for the WAS edited samples. Each assay is repeated in triplicate. Two columns containing the same sample gDNA are required. NTC=non template control. E) MEGA readout performed on WAS edited samples.

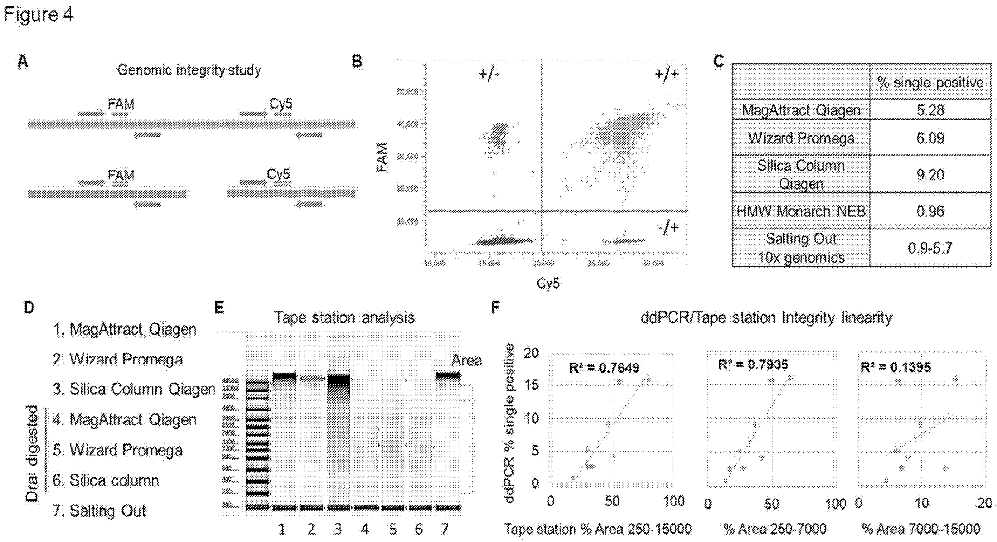

**Figure 4. Genomic integrity study**

A) Schematic of the 1st assay and its relative readout in B. DNA extraction methods can damage the DNA creating fragments of all sizes. MEGA readout will benefit of a more intact genome reducing the background. Extraction methods were compared and tested with genomic integrity assay. Salting out method suggested by 10X genomics and HMW monarch from NEB gave the lowest amount of single positive droplets. C) Tape station capillary electrophoresis was performed on the same samples for a direct comparison with genomic integrity dPCR assay. gDNA was DraI digested for a low molecular weight visual control. The area described in the electrophoretic run was calculate from the signal in the regions from 250bp to 7'000bp and from 7'000bp till 15'000bp to understand the principal factor returning single positive droplets for the dPCR. E) Correlation between dPCR single positive droplets and the signal in the defined area.

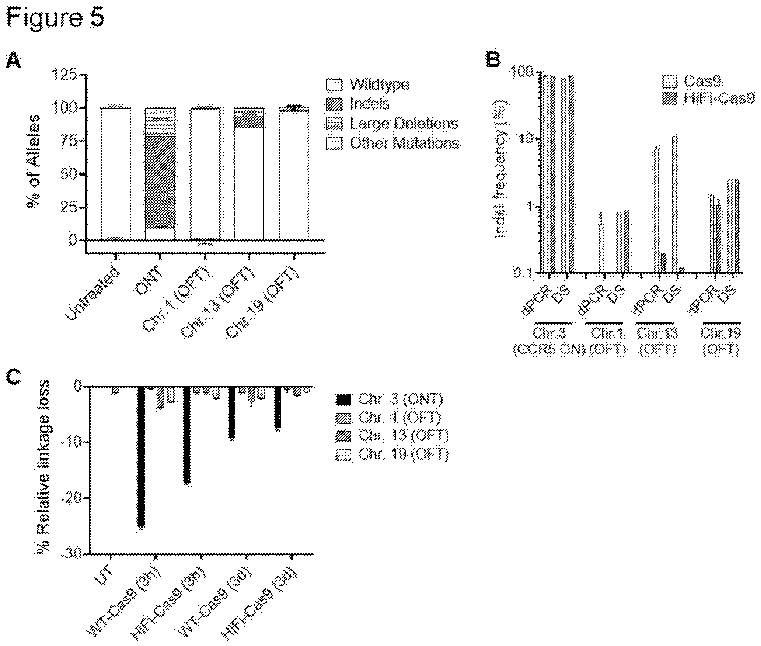

**Figure 5 | CCR5 On-target and known off-target chromosomal aberrations**

**(A)** Summarised overview of the percentage of alleles defined as wildtype, indels, large deletions, and other mutations within the population of cells at the on-target and known off-target loci on chromosome 1, 13, and 19 respectively. **(B)** Comparison of indel frequency induced by Cas9 and high fidelity-Cas9 calculated by digital PCR and deep sequencing. ONT, On-target; OFT, Off-target; DS, Deep sequencing. n=2. mean $\pm$ SD. **(C)** Relative linkage loss comparison among on-and off-targets highlighting that other gross chromosomal aberrations are possible when the cleavage is minimal.

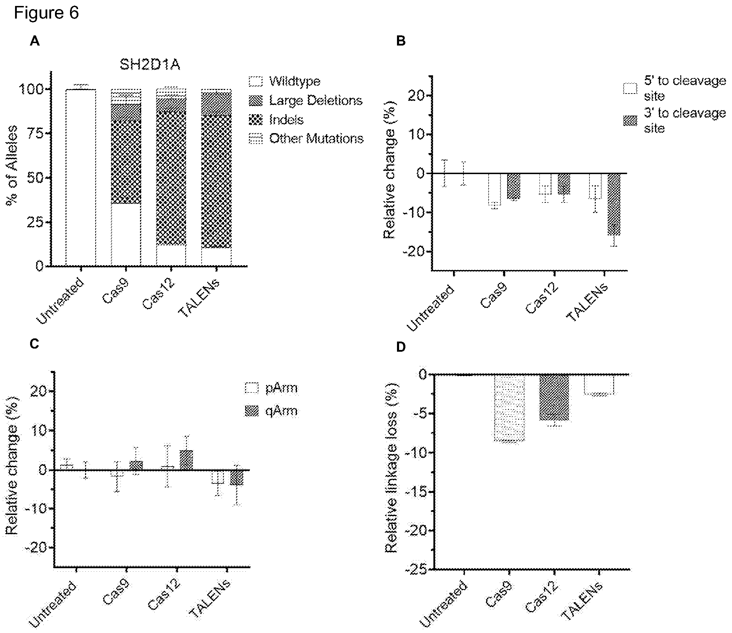

**Figure 6. MEGA summary of Cas9, Cas12, and TALENs edited T cells targeting the SH2D1A locus (3 days post-editing)**

A) Summarised overview of the percentage of alleles defined as wildtype, indels, large deletions, and other undefined mutations within the population of cells. (B) Change of the number amplicon copies 5' and 3' to the cleavage site relative to the untreated cells. (C) Change of the number of copies of p and q arm telomeric amplicons relative to the untreated cells. (D) Relative loss of linked 5' and 3' amplicons compared to the untreated control. WT, Wildtype; UT, Untreated.

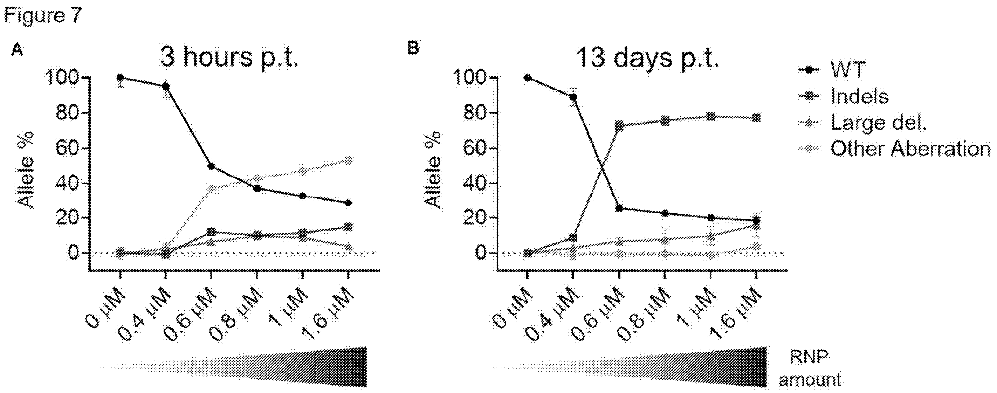

**Figure 7 | Dynamic measurement of mutations through time points and scalar amount of designer nuclease.**

1 million CD34 cells were electroporated with an increasing concentration of Cas9/gRNA RNP (RiboNucleoProtein) targeting the BTK (Bruton's Tyrosine Kinase, involved in XLA immunodeficiency when mutated) gene and the genomic DNA collected at 3 hours (A) and 13 days (B) post treatment (p.t.). The two panels show the different DNA repair dynamics and expose the possibility of reaching a plateau effect of gene editing after 0.6 uM of RNP. n=2. mean ± SD.

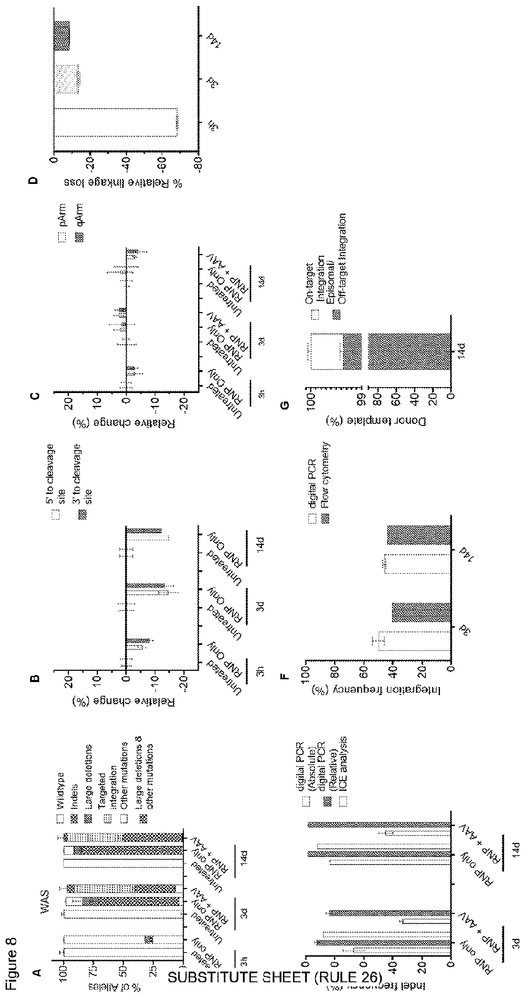

**Figure 8. MEGA summary of Cas9 and Cas9 + AAV edited HSPCs targeting the WAS locus**
A) Summarised overview of the percentage of alleles defined as wildtype, indels, large deletions, and other mutations. B) Change of the number amplicon copies 5' and 3' to the cleavage site relative to the untreated cells. C) Change of the number of copies of p and q arm telomeric amplicons relative to the untreated cells. D) Relative loss of linked 5' and 3' amplicons compared to the untreated control. E) Comparison of indel frequency quantified by the new absolute, relative, and sequencing methods. F) Comparison of dPCR and flow cytometry methods of quantifying AAV integration frequency. G) Quantification of integrated and episomal AAV donor template 2 weeks after transduction.

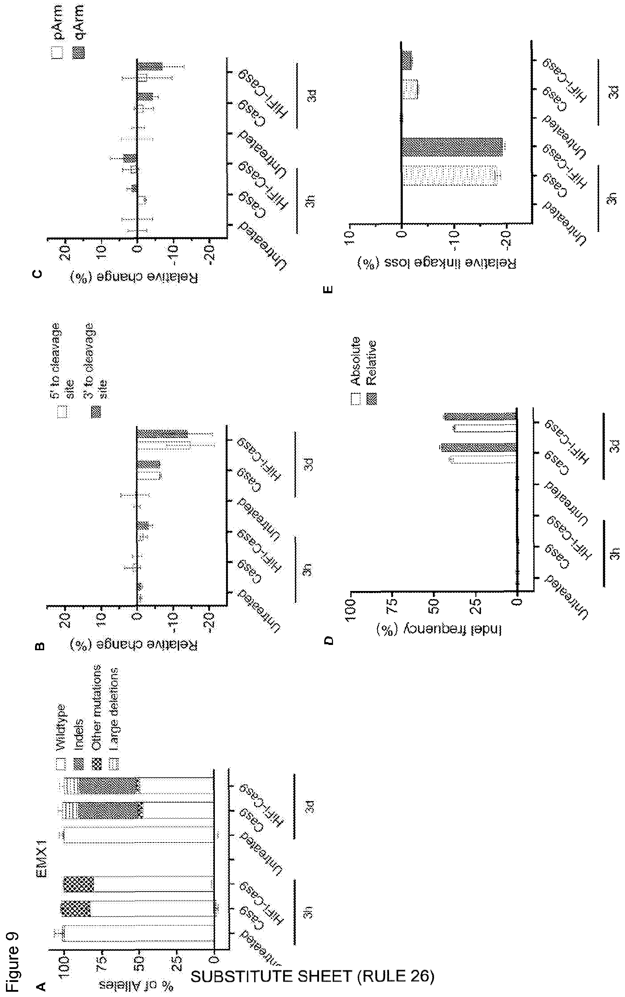

**Figure 9. MEGA summary of Cas9 and HiFi-Cas9 edited HSPCs targeting the EMX1 locus (3h and 3 days post-editing)**

A) Summarised overview of the percentage of alleles defined as wildtype, indels, large deletions, and other mutations. B) Change of the number amplicon copies 5' and 3' to the cleavage site relative to the untreated cells. C) Change of the number of copies of p and q arm telomeric amplicons relative to the untreated cells. D) Comparison of indel frequency quantified by the new absolute method and typically used relative method. E) Relative loss of linked 5' and 3' amplicons compared to the untreated control.

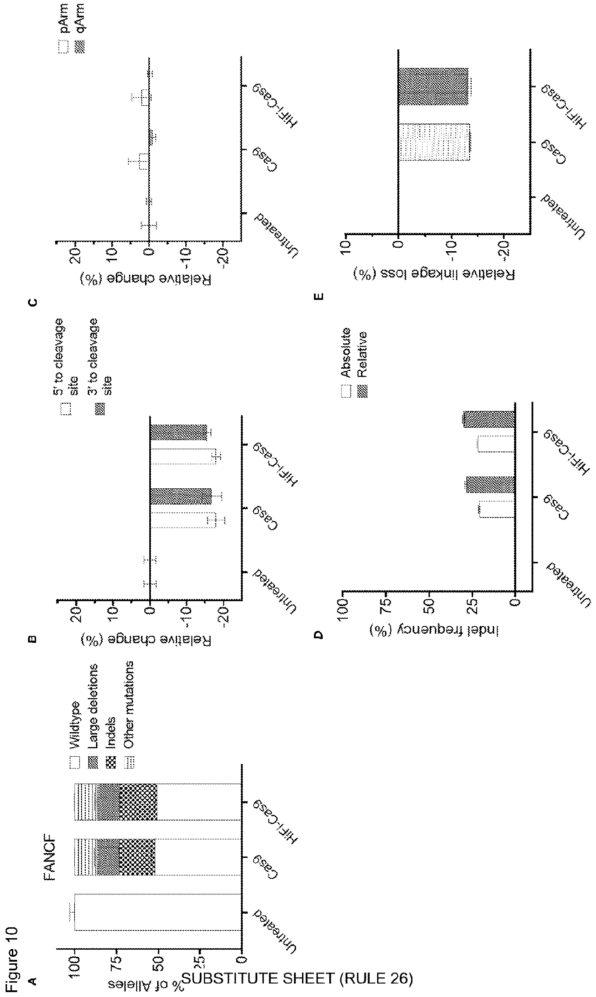

**Figure 10. MEGA summary of Cas9 and HiFi-Cas9 edited HSCPs targeting the FANCF locus (3 days post-editing)**

A) Summarised overview of the percentage of alleles defined as wildtype, indels, large deletions, and other mutations. B) Change of the number amplicon copies 5' and 3' to the cleavage site relative to the untreated cells. C) Change of the number of copies of p and q arm telomeric amplicons relative to the untreated cells. D) Comparison of indel frequency quantified by the new absolute method and typically used relative method. E) Relative loss of linked 5' and 3' amplicons compared to the untreated control.

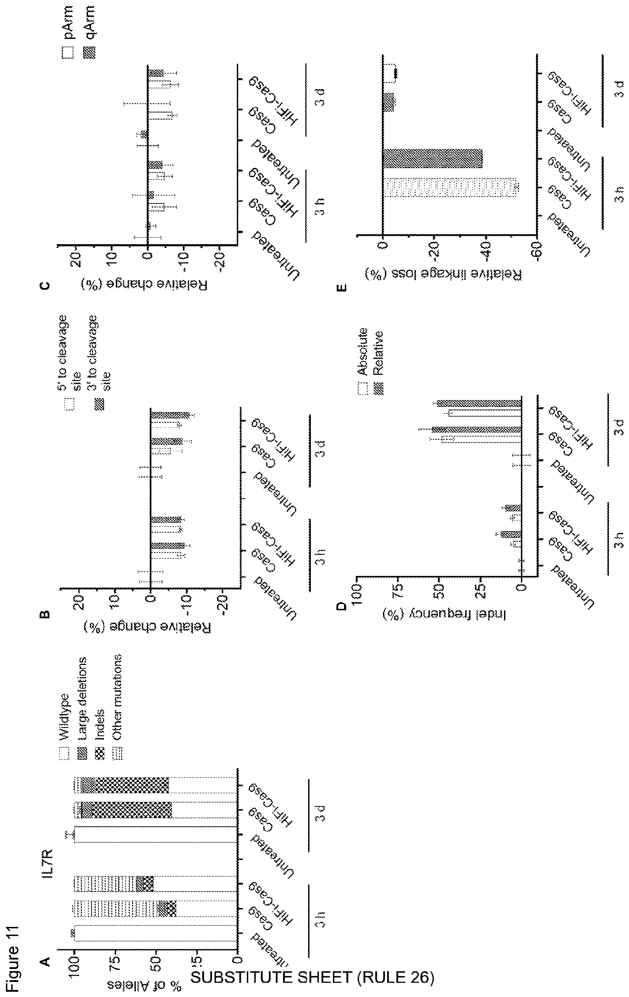

**Figure 11. MEGA summary of Cas9 and HiFi-Cas9 edited HSPCs targeting the IL7R locus (3h and 3 days post-editing)**

A) Summarised overview of the percentage of alleles defined as wildtype, indels, large deletions, and other mutations. B) Change of the number amplicon copies 5' and 3' to the cleavage site relative to the untreated cells. C) Change of the number of copies of p and q arm telomeric amplicons relative to the untreated cells. D) Comparison of indel frequency quantified by the new absolute method and typically used relative method. E) Relative loss of linked 5' and 3' amplicons compared to the untreated control.

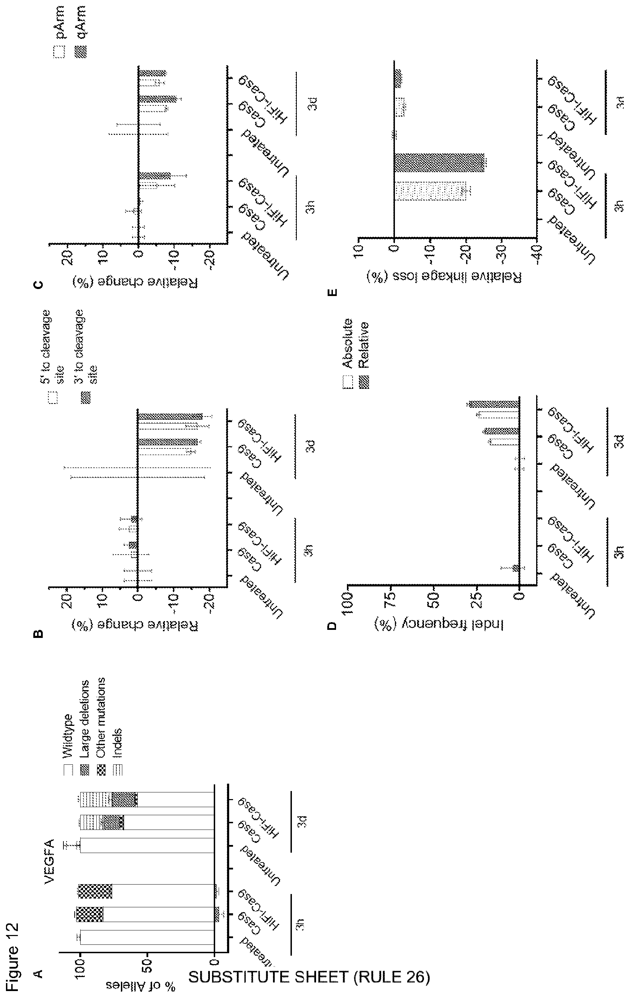

**Figure 12. MEGA summary of Cas9 and HiFi-Cas9 edited HSPCs targeting the VEGFA locus (3h and 3 days post-editing)**

A) Summarised overview of the percentage of alleles defined as wildtype, indels, large deletions, and other mutations. B) Change of the number amplicon copies 5' and 3' to the cleavage site relative to the untreated cells. C) Change of the number of copies of p and q arm telomeric amplicons relative to the untreated cells. D) Comparison of indel frequency quantified by the new absolute method and typically used relative method. E) Relative loss of linked 5' and 3' amplicons compared to the untreated control.

**Examples**

Reagents:

Stilla:

- naica® multiplex PCR MIX 10X (cat.#R10103, Stilla Technologies)

- 3-colour naica® system (cat.#N1001.3, Stilla Technologies)
- Crystal Reader Software (V2.4.0.3, Stilla Technologies)
- Crystal Miner Software (V2.4.0.3, Stilla Technologies)
- Sapphire chips (cat.#C14012-2)
- Opal chips (cat.#C15001-0-15)
- PerfeCTa Multiplex qPCR ToughMix, 250R (cat.# 95147-250)

Bio-Rad:

- QX200 Droplet Digital PCR System (cat.#1864003)
- QXDx Automated Droplet Generator (cat.#17002229)
- QXDx AutoDG Consumable Pack (cat.# 12001922)
- PX1 PCR Plate Sealer. (cat.# 1814000)
- ddPCR Supermix for Probes (No dUTP) (cat.#186-3024)
- Droplet Generation Oil for Probes (cat.#1863005)
- ddPCR™ Droplet Reader Oil (cat.#1863004)
- PCR Plate Heat Seal, foil, pierceable (cat.# 1814040)
- DG8™ Cartridges and Gaskets (cat.#1864007)
- ddPCR™ Buffer Control for Probes (cat.#1863052)
- QuantaSoft Software, version 1.7, (cat.# 1864011)

Qiagen:

- QIAcuity One, 5plex Platform System (cat.#911035)
- QIAcuity Nanoplate 26k 24-well (cat.#250001)
- QIAcuity Nanoplate 8.5k 24-well (cat. #250011)
- QIAcuity Nanoplate 8.5k 96-well (cat. #250021)
- QIAcuity Probe PCR Kit (cat.#250101)

**Primer and probe List:**

The primers and probes listed herein may be used, as an example, in the method and/or compositions of the invention.

Table 1: Target WAS (targeted gRNA sequence: GCAGAAAGCACCATGAGTGG (SEQ ID NO: 1))

| Assay Name | Primer/probe ID | | Notes | Primer/probe sequence (5' ->3') | SEQ ID NO: |
|---|---|---|---|---|---|
| Donor KI-OT | Primers | WAS HR O/O Fwd | | CAGTATTAGTCTGCTGCCTAAGC | (SEQ ID NO: 2) |
| | | WAS HR O/O Rev | | TGATGAGAGATTCCTGGGATG | (SEQ ID NO: 3) |
| | Primers | GFP HR O/O Fwd | | CCGCTTTACTTGTACAGCTC | (SEQ ID NO: 4) |
| | | GFP HR O/O Rev | | AACGAGAAGCGCGATCAC | (SEQ ID NO: 5) |
| | Probes | WAS O/O Probe | HEX | ATGAGCACTGTATTTGTACCTGAACCTCA | (SEQ ID NO: 6) |
| | Probes | HR O/O GFP Probe | FAM | CTGGAGTTCGTGACCGCC | (SEQ ID NO: 7) |

(continued)

| Assay Name | Primer/probe ID | | Notes | Primer/probe sequence (5' ->3') | SEQ ID NO: |
|---|---|---|---|---|---|
| Indels | Primers | WAS Indels Fwd | | CCCCTGGAGGACTTGTTTC | (SEQ ID NO: 8) |
| | | WAS Indels Rev | | CCAGCTCACCAAGCATTTTC | (SEQ ID NO: 9) |
| | Probes | WAS Indels Cut Probe | FAM | CCCACTCATGGTGCTTTCTGCC | (SEQ ID NO: 10) |
| | | WAS Indels Distal Probe | HEX | CATCTCAAAGAGTCGCTGGTTCTCGTG | (SEQ ID NO: 11) |
| Flanking | Primers | WAS Flanking 5' Fwd | | TTCCTGTTCCCTTGCTGCTC | (SEQ ID NO: 12) |
| | | WAS Flanking 5' Rev | | GTCTTCTCTGGCGAGGCTC | (SEQ ID NO: 13) |
| | | WAS Flanking 3' Fwd | | GAGGAGCACCAGCGGTTC | (SEQ ID NO: 14) |
| | | WAS Flanking 3' Rev | | TCAAAGAGTCGCTGGTTCTCG | (SEQ ID NO: 15) |
| | Probes | WAS Flanking 5' Probe | FAM | CGGAAGTTCCTCTTCTTACCCTGCACCC | (SEQ ID NO: 16) |
| | | WAS Flanking 3' Probe | HEX | CAGAACATACCCTCCACCCTCCTCCAG | (SEQ ID NO: 17) |
| LoH | Primers | ChrX pArm Fwd | | CCTGGTAGCTTTCGATGTTGATG | (SEQ ID NO: 18) |
| | | ChrX pArm Rev | | GGCCTGGACCTATCTCAC | (SEQ ID NO: 19) |
| | | ChrX qArm Fwd | | TGTCAATAACAGGCACTTGACAAAC | (SEQ ID NO: 20) |
| | | ChrX qArm Rev | | CCTGAGGCGATGGTGAAAG | (SEQ ID NO: 21) |
| | Probes | ChrX pArm Probe | FAM | CTTTGGTGTCCACCCTCCACCTC | (SEQ ID NO: 22) |
| | | ChrX qArm Probe | HEX | CAAGCATGGAAGCATCTCCCAAGGC | (SEQ ID NO: 23) |

Table 2: Target BTK (targeted gRNA sequence: GATGCTCTCCAGAATCACTG (SEQ ID NO: 234))

| Assay Name | Primer/probe ID | | Notes | Primer/probe sequence (5' ->3') | SEQ ID NO: |
|---|---|---|---|---|---|
| Donor KI-OT | Primers | BTK HR O/O Fwd | | AAGAGGCAGTAAGAAGGGTTC | (SEQ ID NO: 24) |
| | | BTK HR O/O Rev | | CCCATCTCAGACATTGGTCTC | (SEQ ID NO: 25) |
| | Primers | GFP HR O/O Fwd | | CCGCTTTACTTGTACAGCTC | (SEQ ID NO: 26) |
| | | GFP HR O/O Rev | | AACGAGAAGCGCGATCAC | (SEQ ID NO: 27) |
| | Probes | BTK HR O/O Probe | HEX | CGGAATCTGTCTTTCTGGAGGAGGAT | (SEQ ID NO: 28) |
| | Probes | HR O/O GFP Probe | FAM | CTGGAGTTCGTGACCGCC | (SEQ ID NO: 29) |
| Indels | Primers | BTK Indels Fwd | | ACCCCACGTTCAAAGTCATAC | (SEQ ID NO: 30) |
| | | BTK Indels Rev | | CTGGGTCCTCAGGAACTTTC | (SEQ ID NO: 31) |
| | Probes | BTK Indels Cut Probe | FAM | CTATGGCCGCAGTGATTCTGGAG | (SEQ ID NO: 32) |
| | | BTK Indels Distal Probe | HEX | AGGAGAGTTTGTGCACGGTCAAG | (SEQ ID NO:33) |
| Flanking | Primers | BTK Flanking 5' Fwd | | GAACCAAGAGGGATGAGGATT | (SEQ ID NO: 34) |
| | | BTK Flanking 5' Rev | | GTTCACCTGTGTGCTGTTG | (SEQ ID NO: 35) |
| | | BTK Flanking 3' Fwd | | GCATCTTTCTGAAGCGATCC | (SEQ ID NO: 36) |
| | | BTK Flanking 3' Rev | | GTGCACGGTCAAGAGAAAC | (SEQ ID NO: 37) |
| | Probes | BTK Flanking 5' Probe | FAM | TCCTGGGTCCTCAGGAACTTTCATTATCAAC | (SEQ ID NO: 38) |
| | | BTK Flanking 3' Probe | HEX | AACATCACCTCTAAACTTCAAGAAGCGCCT | (SEQ ID NO: 39) |
| LoH | Primers | ChrX pArm Fwd | | CCTGGTAGCTTTCGATGTTGATG | (SEQ ID NO: 40) |
| | | ChrX pArm Rev | | GGCCTGGACCTATCTCAC | (SEQ ID NO: 41) |
| | | ChrX qArm Fwd | | TGTCAATAACAGGCACTTGACAAAC | (SEQ ID NO: 42) |
| | | ChrX qArm Rev | | CCTGAGGCGATGGTGAAAG | (SEQ ID NO: 43) |
| | Probes | ChrX pArm Probe | FAM | CTTTGGTGTCCACCCTCCACCTC | (SEQ ID NO: 44) |
| | | ChrX qArm Probe | HEX | CAAGCATGGAAGCATCTCCCAAGGC | (SEQ ID NO: 45) |

Table 3: Target IL7R (targeted gRNA sequence: TCTCTCAGAATGACAATTCT (SEQ ID NO: 235))

| Assay Name | Primer/probe ID | | Notes | Primer/probe sequence (5' ->3') | SEQ ID NO: |
|---|---|---|---|---|---|
| Donor KI-OT | Primers | IL7R HR O/O Fwd | | AGGCATGTGGGAATTTAGACC | (SEQ ID NO: 46) |
| | | IL7R HR O/O Rev | | AGCTGAGTCATTAGGCTGATG | (SEQ ID NO: 47) |
| | Primers | GFP HR O/O Fwd | | CCGCTTTACTTGTACAGCTC | (SEQ ID NO: 48) |
| | | GFP HR O/O Rev | | AACGAGAAGCGCGATCAC | (SEQ ID NO: 49) |
| | Probes | IL7R HR O/O Probe | HEX | CTTGCTCCTGCAAATTAAGCCCTTTCTC | (SEQ ID NO: 50) |
| | Probes | HR O/O GFP Probe | FAM | CTGGAGTTCGTGACCGCC | (SEQ ID NO: 51) |
| Indels | Primers | IL7R Indels Fwd | | TTATACTTCCCTTGTCTGTGGTTAG | (SEQ ID NO: 52) |
| | | IL7R Indels Rev | | TTAGGGAACTGAATAACCTGAAACC | (SEQ ID NO: 53) |
| | Probes | IL7R Indels Cut Probe | FAM | CAGAATGACAATTCTAGGTACAAC | (SEQ ID NO: 54) |
| | | IL7R Indels Distal Probe | HEX | CCTAGATCTAAGCTTCTCTGTCTTCCTCCC | (SEQ ID NO: 55) |
| Flanking | Primers | IL7R Flanking 5' Fwd | | CCTAGATCTAAGCTTCTCTGTCTTC | (SEQ ID NO: 56) |
| | | IL7R Flanking 5' Rev | | GAGAGAGAGTAGATGTGTGAGC | (SEQ ID NO: 57) |
| | | IL7R Flanking 3' Fwd | | CTTTACTTCAAGTCGTTTCTGGAG | (SEQ ID NO: 58) |
| | | IL7R Flanking 3' Rev | | GTTAGGGAACTGAATAACCTGAAAC | (SEQ ID NO: 59) |
| | Probes | IL7R Flanking 5' Probe | FAM | TCCCTCCCTTCCTCTTACTCTCATTCATT | (SEQ ID NO: 60) |
| | | IL7R Flanking 3' Probe | HEX | TGGCTATGCTCAAAATGGTGAGTCATTTCT | (SEQ ID NO: 61) |
| LoH | Primers | Chr5 pArm Fwd | | CTCCGATGTCATCACCTCAC | (SEQ ID NO: 62) |
| | | Chr5 pArm Rev | | GGATAGACAATGTACCCACTTGG | (SEQ ID NO: 63) |
| | | Chr5 qArm Fwd | | TCTCACCACTGACCAGTTTG | (SEQ ID NO: 64) |
| | | Chr5 qArm Rev | | CGTACAGGATGATGTCCGT | (SEQ ID NO: 65) |
| | Probes | Chr5 pArm Probe | FAM | ACCGTCTTCATTTGCACCTGTGAG | (SEQ ID NO: 66) |
| | | Chr5 qArm Probe | HEX | TGTGTGTGTCCATGTGCGAGG | (SEQ ID NO: 67) |

Table 4: Target CCR5 (targeted gRNA sequence: GTGAGTAGAGCGGAGGCAGG (SEQ ID NO: 236))

| Assay Name | Primer/probe ID | | Notes | Primer/probe sequence (5' ->3') | SEQ ID NO: |
|---|---|---|---|---|---|
| Indels | Primers | CCR5 Indels Fwd | | AGGGCAACTAAATACATTCTAGGAC | (SEQ ID NO: 68) |
| | | CCR5 Indels Rev | | TAGATGTCAGTCATGCTCTTCAG | (SEQ ID NO: 69) |
| | Probes | CCR5 Indels Cut Probe | FAM | CAGCCCGCCTCCTGCCTC | (SEQ ID NO: 70) |
| | | CCR5 Indels Distal Probe | HEX | ATGCACAGGGTGGAACAAGATGGATTATC | (SEQ ID NO: 71) |
| Flanking | Primers | CCR5 Flanking 5' Fwd | | AGGGCAACTAAATACATTCTAGGAC | (SEQ ID NO: 72) |
| | | CCR5 Flanking 5' Rev | | CAGGGCTCCGATGTATAATAATTG | (SEQ ID NO: 73) |
| | | CCR5 Flanking 3' Fwd | | CTTTGGTTTTGTGGGCAAC | (SEQ ID NO: 74) |
| | | CCR5 Flanking 3' Rev | | AGGTAGATGTCAGTCATGCT | (SEQ ID NO: 75) |
| | Probes | CCR5 Flanking 5' Probe | FAM | ATGCACAGGGTGGAACAAGATGGATTATC | (SEQ ID NO: 76) |
| | | CCR5 Flanking 3' Probe | HEX | CTGGTCATCCTCATCCTGATAAACTGC | (SEQ ID NO: 77) |
| LoH | Primers | Chr3 pArm Fwd | | AAGAATCTGCCTGATTCACCTTC | (SEQ ID NO: 78) |
| | | Chr3 pArm Rev | | CTGGCTTACATGGTAGTGTGC | (SEQ ID NO: 79) |
| | | Chr3 qArm Fwd | | CCGGTTCCAGAATCTGAG | (SEQ ID NO: 80) |
| | | Chr3 qArm Rev | | TCCAGATCTTCAGATGGGAC | (SEQ ID NO: 81) |
| | Probes | Chr3 pArm Probe | FAM | GGTAAAGGAGTCCGAGAGATACCCG | (SEQ ID NO: 82) |
| | | Chr3 qArm Probe | HEX | CCTTTGGCATCTCACACAGTGGAAATTC | (SEQ ID NO: 83) |

Table 5: Internal House Keeping Control (Normalisation)/ Genomic integrity*

| Assay Name | Primer/probe ID | | Notes | Primer/probe sequence (5' ->3') | SEQ ID NO: |
|---|---|---|---|---|---|
| DNA Integrity | Primers | DNA Integrity Fwd | | ACTTTGCAAAATGTGGAGACCA | (SEQ ID NO: 84) |
| | | DNA Integrity Rev | | AGACAGATGGTTCAAGCGTG (SEQ ID NO: 85) | (SEQ ID NO: 85) |
| | Probes | DNA Integrity Probe | FAM | TGCAGATAGCACTCTTCACCTGGAGA | (SEQ ID NO: 86) |
| | Primers | Control Fwd | | AAGGAGACTGCAGGCTAC | (SEQ ID NO: 87) |
| | | Control Rev | | GTTGGTGACTGCTGGATTAAG | (SEQ ID NO: 88) |
| | Probes | Control Probe | CY5 or HEX | CAGGGAGGGAAGATCCGGAC | (SEQ ID NO: 89) |
| | *The primer/probes from a flanking assay from another target can be implemented as a second internal reference to help reduce inter-assay variability. | | | | |

Table 6: Target EMX1 (targeted gRNA sequence: GAGTCCGAGCAGAAGAAGAA (SEQ ID NO: 237))

| Assay Name | Primer/probe ID | | Notes | Primer/probe sequence (5' ->3') | SEQ ID NO: |
|---|---|---|---|---|---|
| Indels | Primers | EMX1 Indels Fwd | | CCAGGTGAAGGTGTGG | (SEQ ID NO: 90) |
| | | EMX1 Indels Rev | | GTTGCCCACCCTAGTC | (SEQ ID NO: 91) |
| | Probes | EMX1 Indels Cut Probe | FAM | CTGAGTCCGAGCAGAAGAAGAAGGG | (SEQ ID NO: 92) |
| | | EMX1 Indels Distal Probe | HEX | TGGAGGTGACATCGATGTCCTCC | (SEQ ID NO: 93) |
| Flanking | Primers | EMX1 Flanking 5' Fwd | | CCAGGTGAAGGTGTGG | (SEQ ID NO: 94) |
| | | EMX1 Flanking 5' Rev | | CCTCCTCCAGCTTCTG | (SEQ ID NO: 95) |
| | | EMX1 Flanking 3' Fwd | | ACGAAGCAGGCCAATG | (SEQ ID NO: 96) |
| | | EMX1 Flanking 3' Rev | | GTTGCCCACCCTAGTC | (SEQ ID NO: 97) |
| | Probes | EMX1 Flanking 5' Probe | FAM | TCCAGAACCGGAGGACAAAGTACAAAC | (SEQ ID NO: 98) |
| | | EMX1 Flanking 3' Probe | HEX | TGGAGGTGACATCGATGTCCTCC | (SEQ ID NO: 99) |

(continued)

| Assay Name | Primer/probe ID | | Notes | Primer/probe sequence (5' ->3') | SEQ ID NO: |
|---|---|---|---|---|---|
| LoH | Primers | Chr2 pArm Fwd | | GACCACAACAAGGTACCG | (SEQ ID NO: 100) |
| | | Chr2 pArm Rev | | GGTTGTCATCTGCTCCAC | (SEQ ID NO: 101) |
| | | Chr2 qArm Fwd | | GACGAAGGATGGCAACAG | (SEQ ID NO: 102) |
| | | Chr2 qArm Rev | | CAGTGAGCCAAACGACG | (SEQ ID NO: 103) |
| | Probes | Chr2 pArm Probe | FAM | TCGACCCGCTGGTGTCTTGC | (SEQ ID NO: 104) |
| | | Chr2 qArm Probe | HEX | CATCAAAGGCTCCTCGTTGAGCTCG | (SEQ ID NO: 105) |

Table 7: Target FANCF (targeted gRNA sequence: GGAATCCCTTCTGCAGCACC (SEQ ID NO: 238))

| Assay Name | Primer/probe ID | | Notes | Primer/probe sequence (5' ->3') | SEQ ID NO: |
|---|---|---|---|---|---|
| Indels | Primers | FANCF Indels Fwd | | GGATGTTCCAATCAGTACGC | (SEQ ID NO: 106) |
| | | FANCF Indels Rev | | GATGTGGCGCAGGTAG | (SEQ ID NO: 107) |
| | Probes | FANCF Indels Cut Probe | FAM | CATGGAATCCCTTCTGCAGCACC | (SEQ ID NO: 108) |
| | | FANCF Indels Distal Probe | HEX | CCCAGGTGCTGACGTAGGTAGT | (SEQ ID NO: 109) |
| Flanking | Primers | FANCF Flanking 5' Fwd | | GGATGTTCCAATCAGTACGC | (SEQ ID NO: 110) |
| | | FANCF Flanking 5' Rev | | GCCCTACTTCCGCTTTC | (SEQ ID NO: 111) |
| | | FANCF Flanking 3' Fwd | | CTTCTGGCGGTCTCAAG | (SEQ ID NO: 112) |
| | | FANCF Flanking 3' Rev | | GATGTGGCGCAGGTAG | (SEQ ID NO: 113) |
| | Probes | FANCF Flanking 5' Probe | FAM | CCTTGGAGACGGCGACTCTC | (SEQ ID NO: 114) |
| | | FANCF Flanking 3' Probe | HEX | CCCAGGTGCTGACGTAGGTAGT | (SEQ ID NO: 115) |

(continued)

| Assay Name | Primer/probe ID | | Notes | Primer/probe sequence (5' ->3') | SEQ ID NO: |
|---|---|---|---|---|---|
| LoH | Primers | Chr11 pArm Fwd | | TCCCAGGTGGAGACAG | (SEQ ID NO: 116) |
| | | Chr11 pArm Rev | | CGAACGTGGGTAGCAC | (SEQ ID NO: 117) |
| | | Chr11 qArm Fwd | | TAGGTTTGACTGAGAAGAGCG | (SEQ ID NO: 118) |
| | | Chr11 qArm Rev | | GAGTTCTGGATGACACTGTC | (SEQ ID NO: 119) |
| | Probes | Chr11 pArm Probe | FAM | TGCTGAAGCAGCTACGGCCAG | (SEQ ID NO: 120) |
| | | Chr11 qArm Probe | HEX | CCCAAGATACAGAACAGCCTCTCAGG | (SEQ ID NO: 121) |

Table 8: Target RAG1A (targeted gRNA sequence: GCCTCTTTCCCACCCACCTT (SEQ ID NO: 238))

| Assay Name | Primer/probe ID | | Notes | Primer/probe sequence (5' ->3') | SEQ ID NO: |
|---|---|---|---|---|---|
| Indels | Primers | RAG1A Indels Fwd | | ACATCAGTGGGATATTGATATTGG | (SEQ ID NO: 122) |
| | | RAG1A Indels Rev | | CTTCAGGTGTCTTTTCAAAGGATC | (SEQ ID NO: 123) |
| | Probes | RAG1A Indels Cut Probe | FAM | AGCCTCTTTCCCACCCACCTTG | (SEQ ID NO: 124) |
| | | RAG1A Indels Distal Probe | HEX | CACCCGGAACAGCTTAAATTTCCATTCT | (SEQ ID NO: 125) |
| Flanking | Primers | RAG1A Flanking 5' Fwd | | ACATCAGTGGGATATTGATATTGG | (SEQ ID NO: 126) |
| | | RAG1A Flanking 5' Rev | | ATGCTGGCTGAGGTAC | (SEQ ID NO: 127) |
| | | RAG1A Flanking 3' Fwd | | AGATGAAATTCAGCACCCAC | (SEQ ID NO: 128) |
| | | RAG1A Flanking 3' Rev | | CTTCAGGTGTCTTTTCAAAGGATC | (SEQ ID NO: 129) |
| | Probes | RAG1A Flanking 5' Probe | FAM | TGAGAACAATGAAAACAAGTCATATTAAGACC | (SEQ ID NO: 130) |
| | | RAG1A Flanking 3' Probe | HEX | CACCCGGAACAGCTTAAATTTCCATTCT | (SEQ ID NO: 131) |

(continued)

| Assay Name | Primer/probe ID | | Notes | Primer/probe sequence (5' ->3') | SEQ ID NO: |
|---|---|---|---|---|---|
| LoH | Primers | Chr11 pArm Fwd | | TCCCAGGTGGAGACAG | (SEQ ID NO: 132) |
| | | Chr11 pArm Rev | | CGAACGTGGGTAGCAC | (SEQ ID NO: 133) |
| | | Chr11 qArm Fwd | | TAGGTTTGACTGAGAAGAGCG | (SEQ ID NO: 134) |
| | | Chr11 qArm Rev | | GAGTTCTGGATGACACTGTC | (SEQ ID NO: 135) |
| | Probes | Chr11 pArm Probe | FAM | TGCTGAAGCAGCTACGGCCAG | (SEQ ID NO: 136) |
| | | Chr11 qArm Probe | HEX | CCCAAGATACAGAACAGCCTCTCAGG | (SEQ ID NO: 137) |

Table 9: Target RAG1B (targeted gRNA sequence: GACTTGTTTTCATTGTTCTC (SEQ ID NO: 239))

| Assay Name | Primer/probe ID | | Notes | Primer/probe sequence (5' ->3') | SEQ ID NO: |
|---|---|---|---|---|---|
| Indels | Primers | RAG1B Indels Fwd | | GTATAAGTAACCATAAACACTGTCAG | (SEQ ID NO:138) |
| | | RAG1B Indels Rev | | CTGAAAATTTAATATGTGGGTGCTG | (SEQ ID NO: 139) |
| | Probes | RAG1B Indels Cut Probe | FAM | ATGACTTGTTTTCATTGTTCTCAGGTACCTC | (SEQ ID NO: 140) |
| | | RAG1B Indels Distal Probe | HEX | CATCTGGGGCAGAACTGAGTCCC | (SEQ ID NO: 141) |
| Flanking | Primers | RAG1B Flanking 5' Fwd | | GTATAAGTAACCATAAACACTGTCAG | (SEQ ID NO: 142) |
| | | RAG1B Flanking 5' Rev | | ACCAATATCAATATCCCACTGATG | (SEQ ID NO: 143) |
| | | RAG1B Flanking 3' Fwd | | GCAGCCTCTTTCCCAC | (SEQ ID NO: 144) |
| | | RAG1B Flanking 3' Rev | | CTGAAAATTTAATATGTGGGTGCTG | (SEQ ID NO: 145) |
| | Probes | RAG1B Flanking 5' Probe | FAM | CATGTTAGGTGCTGATCATAGAGTTATTTCCTC | (SEQ ID NO: 146) |
| | | RAG1B Flanking 3' Probe | HEX | CATCTGGGGCAGAACTGAGTCCC | (SEQ ID NO: 147) |

(continued)

| Assay Name | Primer/probe ID | | Notes | Primer/probe sequence (5' ->3') | SEQ ID NO: |
|---|---|---|---|---|---|
| LoH | Primers | Chr11 pArm Fwd | | TCCCAGGTGGAGACAG | (SEQ ID NO: 148) |
| | | Chr11 pArm Rev | | CGAACGTGGGTAGCAC | (SEQ ID NO:149) |
| | | Chr11 qArm Fwd | | TAGGTTTGACTGAGAAGAGCG | (SEQ ID NO:150) |
| | | Chr11 qArm Rev | | GAGTTCTGGATGACACTGTC | (SEQ ID NO: 151) |
| | Probes | Chr11 pArm Probe | FAM | TGCTGAAGCAGCTACGGCCAG | (SEQ ID NO: 152) |
| | | Chr11 qArm Probe | HEX | CCCAAGATACAGAACAGCCTCTCAGG | (SEQ ID NO: 153) |

Table 10: Target VEGFA (targeted gRNA sequence: GGTGAGTGAGTGTGTGCGTG (SEQ ID NO: 240))

| Assay Name | Primer/probe ID | | Notes | Primer/probe sequence (5' ->3') | SEQ ID NO: |
|---|---|---|---|---|---|
| Indels | Primers | VEGFA Indels Fwd | | CACCACAGGGAAGCTG | (SEQ ID NO: 154) |
| | | VEGFA Indels Rev | | GAGAGGGACACACAGATC | (SEQ ID NO: 155) |
| | Probes | VEGFA Indels Cut Probe | FAM | TGGAATCCTGGAGTGACCCCTG | (SEQ ID NO: 156) |
| | | VEGFA Indels Distal Probe | HEX | TGGAATCCTGGAGTGACCCCTG | (SEQ ID NO: 157) |
| Flanking | Primers | VEGFA Flanking 5' Fwd | | CACCACAGGGAAGCTG | (SEQ ID NO: 158) |
| | | VEGFA Flanking 5' Rev | | CACACGTCCTCACTCTC | (SEQ ID NO: 159) |
| | | VEGFA Flanking 3' Fwd | | TTGGAGCGGGGAGAAG | (SEQ ID NO: 160) |
| | | VEGFA Flanking 3' Rev | | GAGAGGGACACACAGATC | (SEQ ID NO: 161) |
| | Probes | VEGFA Flanking 5' Probe | FAM | TGAATGGAGCGAGCAGCGTCTTC | (SEQ ID NO: 162) |
| | | VEGFA Flanking 3' Probe | HEX | TGGAATCCTGGAGTGACCCCTG | (SEQ ID NO: 163) |

(continued)

| Assay Name | Primer/probe ID | | Notes | Primer/probe sequence (5' ->3') | SEQ ID NO: |
|---|---|---|---|---|---|
| LoH | Primers | Chr6 pArm Fwd | | GAGCCAGTCGAAGAGC | (SEQ ID NO: 164) |
| | | Chr6 pArm Rev | | GAGCACGTTGAGAGATCTC | (SEQ ID NO: 165) |
| | | Chr6 qArm Fwd | | GAGAAGGCACTGCCAC | (SEQ ID NO: 166) |
| | | Chr6 qArm Rev | | CTACACCACGCGGAAC | (SEQ ID NO: 167) |
| | Probes | Chr6 pArm Probe | FAM | CAGGACACCTCTGGCGTTCCC | (SEQ ID NO: 168) |
| | | Chr6 qArm Probe | HEX | CCATCTGCACCACACCTATCACGC | (SEQ ID NO: 169) |

Table 11: Target SH2D1A (SAP) (targeted gRNA sequence: ATACACAGCCACTGCGTCCA (SEQ ID NO: 241))

| Assay Name | Primer/probe ID | | Notes | Primer/probe sequence (5' ->3') | SEQ ID NO: |
|---|---|---|---|---|---|
| Indels | Primers | SAP Indels Fwd | | GTTGACTTGTGCCTGGC | (SEQ ID NO: 170) |
| | | SAP Indels Rev | | AAATAGCTGCCATCCAGC | (SEQ ID NO: 171) |
| | Probes | SAP Indels Cut Probe | FAM | CCACTGCGTCCATGGCCTG | (SEQ ID NO: 172) |
| | | SAP Indels Distal Probe | HEX | CGAGAAGCTCCTGCTTGCCAC | (SEQ ID NO: 173) |
| Flanking | Primers | SAP Flanking 5' Fwd | | GTTGACTTGTGCCTGGC | (SEQ ID NO: 174) |
| | | SAP Flanking 5' Rev | | CGAGGAGGAGAACTGTG | (SEQ ID NO: 175) |
| | | SAP Flanking 3' Fwd | | GCAAAATCAGCAGGGAAAC | (SEQ ID NO: 176) |
| | | SAP Flanking 3' Rev | | AAATAGCTGCCATCCAGC | (SEQ ID NO: 177) |
| | Probes | SAP Flanking 5' Probe | FAM | AGGGAGATGCCGCTGCTACTG | (SEQ ID NO: 178) |
| | | SAP Flanking 3' Probe | HEX | CGAGAAGCTCCTGCTTGCCAC | (SEQ ID NO: 179) |

(continued)

| Assay Name | Primer/probe ID | | Notes | Primer/probe sequence (5' ->3') | SEQ ID NO: |
|---|---|---|---|---|---|
| LoH | Primers | ChrX pArm Fwd | | CCTGGTAGCTTTCGATGTTGATG | (SEQ ID NO: 180) |
| | | ChrX pArm Rev | | GGCCTGGACCTATCTCAC | (SEQ ID NO: 181) |
| | | ChrX qArm Fwd | | TGTCAATAACAGGCACTTGACAAAC | (SEQ ID NO: 182) |
| | | ChrX qArm Rev | | CCTGAGGCGATGGTGAAAG | (SEQ ID NO: 183) |
| | Probes | ChrX pArm Probe | FAM | CTTTGGTGTCCACCCTCCACCTC | (SEQ ID NO: 184) |
| | | ChrX qArm Probe | HEX | CAAGCATGGAAGCATCTCCCAAGGC | (SEQ ID NO: 185) |

Table 12: Target CCR5 Off-targets

| Assay Name | Primer/probe ID | | Notes | Primer/probe sequence (5' ->3') | SEQ ID NO: |
|---|---|---|---|---|---|
| Indels | Primers | CCR5 Chr1_OFT Indels Fwd | | GCACCGGACAAGAGAAG | (SEQ ID NO: 186) |
| | | CCR5 Chr1_OFT Indels Rev | | CCTGCAAAAGAGGGCTG | (SEQ ID NO: 187) |
| | Probes | CCR5 Chr1_OFT Indels Cut Probe | FAM | TAACTGGGTAGAAGGAGGCAGGG | (SEQ ID NO: 188) |
| | | CCR5 Chr1_OFT Indels Distal Probe | HEX | ATTCCGGAAGAGAGATTAGAGGCAGGG | (SEQ ID NO: 189) |
| | Primers | CCR5 Chr13_OFT Indels Fwd | | TGTTAAAAAGTGTAACAGAGCTGG | (SEQ ID NO: 190) |
| | | CCR5 Chr13_OFT Indels Rev | | CCATGGTGTAATGGAACCATTC | (SEQ ID NO: 191) |
| | Probes | CCR5 Chr13_OFT Indels Cut Probe | FAM | AAGGAGGAGAGCGGAGGCAG | (SEQ ID NO: 192) |
| | | CCR5 Chr13_OFT Indels Distal Probe | HEX | TGTCTCACAGGCACAGCACAGC | (SEQ ID NO: 193) |
| | Primers | CCR5 Chr19_OFT Indels Fwd | | TGAGGACAGCTACTCTGAAATC | (SEQ ID NO: 194) |
| | | CCR5 Chr19_OFT Indels Rev | | CGGCTGAAGCATCTTTTCC | (SEQ ID NO: 195) |

(continued)

| Assay Name | Primer/probe ID | | Notes | Primer/probe sequence (5' ->3') | SEQ ID NO: |
|---|---|---|---|---|---|
| | Probes | CCR5 Chr19_OFT Indels Cut Probe | FAM | CCGAGTAGGAGAGGAGGCAGGA | (SEQ ID NO: 196) |
| | | CCR5 Chr19_OFT Indels Distal Probe | HEX | CTGCCCCCTTGCGAGTTTCAC | (SEQ ID NO: 197) |
| Flanking | Primers | CCR5 Chr1_OFT Flanking 5' Fwd | | ACTACTAACAAATGCACAGACAGG | (SEQ ID NO: 198) |
| | | CCR5 Chr1_OFT Flanking 5' Rev | | GATCGCTTATTTCGCAGCTC | (SEQ ID NO: 199) |
| | | CCR5 Chr1_OFT Flanking 3' Fwd | | CTCCAGCTGTTTGCATGAATC | (SEQ ID NO: 200) |
| | | CCR5 Chr1_OFT Flanking 3' Rev | | ACTTCTCCCGGAATTCACAG | (SEQ ID NO: 201) |
| | Probes | CCR5 Chr1_OFT Flanking 5' Probe | FAM | AC CCA GTT GA AAAGAC CCA GTT GC | (SEQ ID NO: 202) |
| | | CCR5 Chr1_DFT Flanking 3' Probe | HEX | CCTCCTCAGGCACTAGAGCTTCC | (SEQ ID NO: 203) |
| | | CCR5 Chr13_OFT Flanking 5' Fwd | | CCCACCAACAACAAAGTGA | (SEQ ID NO: 204) |
| | | CCR5 Chr13_OFT Flanking 5' Rev | | CAGATTCTGGCACTTGCTC | (SEQ ID NO: 205) |
| | | CCR5 Chr13_OFT Flanking 3' Fwd | | TTATAGGTCCCAAACTGCCAC | (SEQ ID NO: 206) |
| | | CCR5 Chr13_OFT Flanking 3' Rev | | TGTTATCCTTCACCATCCACTC | (SEQ ID NO: 207) |
| | | CCR5 Chr13_OFT Flanking 5' Probe | FAM | AGTGCCAGTATTTTCATCCTATGTGCCA | (SEQ ID NO: 208) |
| | | CCR5 Chr13_OFF Flanking 3' Probe | HEX | CA TCA GAG GG ATC AGTCTA GGG ACT AC | (SEQ ID NO: 209) |

(continued)

| Assay Name | Primer/probe ID | | Notes | Primer/probe sequence (5' ->3') | SEQ ID NO: |
|---|---|---|---|---|---|
| | | CCR5 Chr19_OFT Flanking 5' Fwd | | ACGTGTCGAGGAAGTTTGTC | (SEQ ID NO: 210) |
| | | CCR5 Chr19_OFT Flanking 5' Rev | | CAGAAACAGACTGCCTCCT | (SEQ ID NO: 211) |
| | | CCR5 Chr19_OFT Flanking 3' Fwd | | CTGGCAGGAAAAGATGCTTC | (SEQ ID NO: 212) |
| | | CCR5 Chr19_OFT Flanking 3' Rev | | GGAGTCAAAGTCATGCACAG | (SEQ ID NO: 213) |
| | | CCR5 Chr19_OFT Flanking 5' Probe | FAM | CC CAC GGA AG ACAGGC AGG T | (SEQ ID NO: 214) |
| | | CCR5 Chr19_OFF Flanking 3' Probe | HEX | CG CAG TGA TG GGC AAAGGC TA | (SEQ ID NO: 215) |

Table 13: Target NR3C1 (targeted gRNA sequence: TCCAAAGAATCATTAACTCC (SEQ ID NO: 242))

| Assay Name | Primer/probe ID | | Notes | Primer/probe sequence (5' ->3') | SEQ ID NO: |
|---|---|---|---|---|---|
| Indels | Primers | NR3C1 Indels Fwd | | GAGGGTGAAGACGCAG | (SEQ ID NO: 216) |
| | | NR3C1 Indels Rev | | TTTGATTCGGAGTTAACTAAAAGGTTC | (SEQ ID NO: 217) |
| | Probes | NR3C1 Indels Cut Probe | FAM | CTCTACCAGGAGTTAATGATTCTTTG | (SEQ ID NO: 218) |
| | | NR3C1 Indels Distal Probe | HEX | CTCTACCAGGAGTTAATGATTCTTTGGAGTCC | (SEQ ID NO: 219) |
| Flanking | Primers | NR3C1 Flanking 5' Fwd | | GAGGGTGAAGACGCAG | (SEQ ID NO: 220) |
| | | NR3C1 Flanking 5' Rev | | GTGTGCTTGCTCAGGAG | (SEQ ID NO: 221) |
| | | NR3C1 Flanking 3' Fwd | | GGGAAAACATCATAAGCTCTAAAGTC | (SEQ ID NO: 222) |
| | | NR3C1 Flanking 3' Rev | | TTTGATTCGGAGTTAACTAAAAGGTTC | (SEQ ID NO: 223) |
| | Probes | NR3C1 Flanking 5' Probe | FAM | CCTTCACAGTAGCTCCTCCTCTTAGGG | (SEQ ID NO: 224) |
| | | NR3C1 Flanking 3' Probe | HEX | CAAGCTGCCTCTTACTAATCGGATCAGGAAG | (SEQ ID NO: 225) |

Table 14: BTK, IL7R, WAS AAV HR integration (In/Out)

| Assay Name | Primer/probe ID | | Notes | Primer/probe sequence (5' ->3') | SEQ ID NO: |
|---|---|---|---|---|---|
| Homologous recombination (Targeted integration; In/-Out) | Primers | AAV_HR_IO_Fwd | | GAGTAGGTGTCATTCTATTCTGG | (SEQ ID NO: 226) |
| | | BTK_AAV_HR_IO_Rev | | TTGCAAATTTCTTCAAATCTGCTG | (SEQ ID NO: 227) |
| | | WAS_AAV_HR_IO_Rev | | AGGCAGCAGACTAATACTG | (SEQ ID NO: 228) |
| | | IL7R_AAV_HR_IO_Rev | | TCTAAATTCCCACATGCCTC | (SEQ ID NO: 229) |
| | | Reference_Fwd | | GGATCTCCATTTTACACATAAGAC | (SEQ ID NO: 230) |
| | | Reference_Rev | | GTTGGTGACTGCTGGATTAAG | (SEQ ID NO: 231) |
| | Probes | AAV_HR_IO Probe | HEX | AGGATTGGGAAGAGAATAGCAGGCATG | (SEQ ID NO: 232) |
| | | Reference Probe | FAM | CAGGGAGGGAAGATCCGGAC | (SEQ ID NO: 233) |

**Assay design**

Optimal primer and probe design should follow standard qPCR/dPCR rules particularly those regarding specificity, secondary structures, %GC content, primer dimers among multiplexed assays, melting temperatures (Tm), and the presence of single nucleotide polymorphisms (SNPs). All assays should return a robust signal using the same PCR program parameters with a clear separation between the negative and positive populations. Probes running in multiplex should have a distinct fluorophore compatible with the relative device recommendations.

The Tm of the primers should be within the same temperature range, while the probes should be 5-15 Tm degrees higher than the primers. Difficult sequences may also require a probe with the Minor Groove Binding (MGB) or locked nucleic acid (LNA) modification domain to increase the annealing temperature and improve the positive signal intensity over the background.

1st Genomic Integrity/reference control assay:
Two pairs of primers should be designed to obtain the same amplicon length of ca. 60-120 bp. The distance between the two assays should be of ca. 150-250 bp. The target of this assay should ideally be located on a chromosome different to that of the chromosome targeted for nuclease cleavage. (Figure 1C).

2nd Indels assay:
One pair of primers should be designed surrounding the cleavage point with two probes designed to target two loci in the region amplified by the primers. One of them should be placed directly across the cleavage site (cleavage probe) whilst the other probe should be placed at least 20 or 25 bp distant from it (distal probe).

3rd Flanking assays:
Two pairs of primers should be designed 5' and 3' of the cleavage site leaving a space of at least 20 bp from the cleavage site. The distance between the 5' assay forward primer and the 3' reverse primer should be as short as possible.

4th Loss of heterozygosity assay:
Two assays should be designed in sub-telomeric regions of the targeted chromosome 5' and 3' of the cleavage site and ideally 2-10 Mb distant from the chromosomal ends.

5th Targeted integration "Donor KI-OT" strategy assay:
One assay should be designed upstream and adjacent to the donor homology regions (HA), while the other assay is located inside the donor cassette in a specific sequence (DN). In the case of a donor introducing a single mismatched nucleotide, a further probe specificity test is advised (MGB probes would better distinguish a single nucleotide polymorphism). The distance between the assays should as short as possible.

Alternative 5th Targeted integration "In/Out" strategy assay:
The In/Out targeted integration strategy requires a pair of primers and one probe that are specific for the integrated cassette in the targeted locus. One primer should be designed inside the donor cassette and the other outside the donor cassette. In case of short donor cassettes, the probe can be the only specific element recognising the donor sequence.
The distance between the primers should be as short as possible. A control assay should be designed far from the cleavage site or preferentially on another allele and it should match the In/Out amplicon length for a more precise quantification.

## dPCR reaction protocol

Around 10-100ng gDNA (30-50ng range for diploid human genomic DNA) per well. This amount typically generates the required number of droplets. The final concentration of primers and probes is 1 $\mu$M and 250 nM, respectively.
All assays should be performed in duplicate or in triplicate to average the final readout and to possibly remove wells that resulted in a technical failure.
The genomic DNA needs to be mixed in the master mix and only then, aliquoted in the several tubes that will receive the different combinations of primers and probes. This will decrease the pipetting variability among the different assays (with 3 colour and the internal control assay this would not be strictly required).
An untreated (UT) sample should be also analysed alongside edited samples to normalize the inter-assay data fluctuations. The genomic DNA should possibly be from the same donor to control for person-to-person variations and/or other variabilities.
To avoid formation of double positive droplets by chance and to reduce the impact of the normalization for genomic integrity, the amount of sample DNA should result in no more than 20% of positive droplets for the assays of interest.

## *Mathematical approach:*

Quantifying the mutation events in accordance with the invention herein may be determined and analysed according to the following mathematical principles.
The quantifications given by the digital droplet polymerase chain reaction (dPCR) machine (copies/ul) may be normalised twice for all the assays. Three colour dPCR, carrying the house-keeping internal control in multiplex, allows a more precise normalization. For two colour dPCR, the control assay may be carried out in another well.
Normalization over the control assay:

$$Norm1 = \frac{(copies/ul)Assay\_n}{(copies/ul)Assay\_Control}$$

Normalization over the relative normalized UT assay:

$$Norm2 = \frac{Norm1\_x}{Norm1\_ntc}$$

This double normalization allows for the removal of the inter- and intra-assay variabilities caused by different factors such as variable quantities of reagents or assay lengths.

### Loss of heterozygosity

Loss of heterozygosity (LOH) can be calculated utilizing the Norm2 ratios of the relative assays designed in the sub-telomeric p and q regions:

$$LoH\% = (Norm2 - 1) * 100$$

This parameter will indicate the gain or the loss of copies of the two chromosomal portions studied. This parameter may be plotted separately from the other parameters since an allele with a LOH can still retain the edited sequence.

### INDELS

Small indels can be calculated from the Indels assays formed by the "cut" and "distal" probe.

$$small\ indels\% = (Norm2_{cut} - Norm2_{distal}) * 100$$

$$WildType\% = Norm2_{cut} * 100$$

The "Small indels%" parameter should be negative indicating the loss of copies respect to the distal probe. When plotting, the absolute value may be provided.

When this assay is utilized, and considering the UT control sample and a control assay, it is possible to also quantify other chromosomal mutations% (accounting mainly for large deletions, insertions and translocations) data:

$$Mutations\% = (Norm2_{distal} - 1) * 100$$

The "Small indels%" and "Mutations%" parameters should be negative indicating the loss of copies relative to the controls. When plotting the absolute value may be provided. Those parameters are already normalised for the amount of alleles, in the case of a X assay in XY context no other normalizations may be required and the values will reflect the percentage copies with respect to the original allelic number.

When this assay is utilized per se, and not in the MEGA context, it can provide relative information such as the relative percentage of small indels:

$$rel\ small\ indels\% = \left(1 - \frac{\left(\frac{copies}{ul}\right)Assay_{cut}}{\left(\frac{copies}{ul}\right)Assay_{distal}}\right) \times 100$$

### Genomic integrity

Calculate base distance from Forward primer 5' base of Channel 1(ch1) assay to the Forward primer 5' base of channel2 (ch2) assay= *DistAssay1*.

Calculate base distance from Reverse primer 5' base of Channel 1(ch1) assay to the Reverse primer 5' base of channel2 (ch2) assay= *DistAssay2*.

Calculate the number of single positive droplets ($D_{ch\#}$) for the genomic integrity assays obtained from the dPCR and calculate the following ratio with all the relative positive droplets ($D^{ch\#}_{all}$):

$$\%D_{ch1} = \left( \frac{D_{ch1}}{D_{ch1all}} \right)$$

$$\%D_{ch2} = \left( \frac{D_{ch2}}{D_{ch2all}} \right)$$

Calculate the likelihood of breaks per base pair in order to estimate the amount of breaks of a given length:

$$DNABreaks_{chance1}/bp = \left( \frac{\%D_{ch2}}{Dist\,Assay1} \right)$$

$$DNABreaks_{chance2}/bp = \left( \frac{\%D_{ch1}}{Dist\,Assay2} \right)$$

$$DNABreaks_{chance_{avg}}/bp = ((DNABreaks\_chance1)/bp) + (DNABreaks\_chance2/bp))/2$$

This value may be utilized in the Flanking assays and the Homologous Recombination (HR) assays to account for the genomic integrity by multiplying this value to the assays distance.

### *Homologous recombination/targeted insertion/episomal/off-targeted/random insertion.*

Depending on the sequence complexity and the strategy utilized for the targeted integration, it is possible to design two different strategies to calculate the targeted insertion.

*In/out strategy.* No possibility to check for the episomal/randomly integrated donor DNA. No need for normalizations (in/out design with independent control assay of the same length). Normalization is required if the control and reference chromosomes amounts are not equal (e.g.,, X assayed in XY context).

$$Targeted\ insertion\% = \left( \frac{\left( \frac{copies}{ul} \right) Assay_{onsert}}{\left( \frac{copies}{ul} \right) Assay_{control}} \right) * 100$$

*Donor KI-OT strategy.* Needs genomic integrity normalization (Donor KI-OT design). Report the average distance between the two HR assays as similarly calculated for the genomic integrity assay. Here we describe the assay designed on the donor as *DN* and the one outside the homology arm as *HA.*

$$OutDroplets_{HA}^i = DNABreaks_{chance_{avg}}/bp * DistAssay_{DNFar} * droplets_{HA_{all}}^i$$

$$OutDroplets_{DN}^i = DNABreaks_{chance_{avg}}/bp * DistAssay_{HARse} * droplets_{DN_{all}}^i$$

In "OutDroplets", the number of droplets that are on average more likely to be single positive because of the genomic fragmentation rather than double positive can be estimated. The OutDroplets number can be used to normalize the single positive droplets ($nD_{DN}$) from the DN assay that is supposed to be read as double positive.

The linkage between the DN assay and the HA assay can be calculated as described in Regan JF et al. (PlosOne 2013. doi:10.1371/journal.pone.0118270) with some modifications described herein.

DNABreaks$^{chance}_{avg}$= Parameter calculated from the genomic integrity assay

DistAssay$_{DNFor}$= Distance in bases calculated from Forward primer 5' base of the DN assay to the Forward primer 5' base of the HA' assay

DistAssay$_{HARev}$= Distance in bases calculated from Reverse primer 5' base of the DN' assay to the Reverse primer 5' base of the HA' assay

Droplets HA'all= Number of all positive droplets (single and double positive) for the HA' assay

Droplets DN'all= Number of all positive droplets (single and double positive) for the DN' assay

$$nD_{DN} = D_{DN} - OutDroplets_{DN}$$

$$nD_{HA} = D_{HA} - OutDroplets_{HA}$$

$D_{DN}$= Number of single positive droplets for the DN assay

$D_{HA}$= Number of single positive droplets for the HA assay

$$nD_{empty} = D_{empty} + D_{control} + OutDroplets_{min value}$$

$$D_{ch} = nD_{DN} * \frac{nD_{HA}}{nD_{empty}}$$

$D_{empty}$= Number of negative droplets

$D_{control}$= Number of single positive droplets from the control assay (when 3 colours utilized)

OutDroplets$_{min value}$= The minimal value taken from either the OutDroplets$_{DN}$ or OutDroplets$_{HA}$

$D_{ch}$= Droplets that are double positive by chance

$$D_{notDNHA} = nD_{empty} + nD_{DN} + nD_{HA} + D_{ch} + D_{control}$$

$$\lambda DNHA = \ln(D_{tot}) - \ln(D_{notDNHA})$$

$$\lambda DN = \ln(D_{tot}) - \ln(D_{notDN})$$

$$\lambda HA = \ln(D_{tot}) - \ln(D_{notHA})$$

$$\%linkage = \lambda DNHA / \lambda HA * 100$$

$$\lambda control = \ln(D_{tot}) - \ln(D_{notcontrol})$$

$$\%targeted\ integration = \lambda DNHA / \lambda control * 100$$

$D_{notDNHA}$= Droplets that are not DN and HA double positive

$\lambda$DNHA= Average copies per droplets of double positive DN/HA assays

$\lambda$DN= Average copies per droplets of DN assay

$\lambda$HA= Average copies per droplets of double positive HA assay

$D_{tot}$= Total amount of droplets

$D_{notDN}$= Number of negative droplets for the DN assay (single and double positive)

$D_{notHA}$= Number of negative droplets for the HA assay (single and double positive)

*%linkage* will return the linkage value among the two assays but other mutations will affect the HA assays (e.g., large deletions). The normalized value of targeted integration% of at the targeted site will be better estimated with the ratio between the double positive copies over the copies of the unrelated control assay. The results will represent the percent of alleles with targeted integration over the total alleles.

$$NormLinkageLoss = (\%LinkageFlankingTreated / \%LinkageGenomicIntegrityTreated / \%LinkageFlankingTreated / \%LinkageGenomicIntegrityTreated /) - 1) * 100$$

This Parameter will represent the category of the "other aberrations" representing mutations such as translocations, open ends, inversions, chromothripsis repairs.

In this case two main factors can be considered:

1) The male genome is composed by XY alleles. Everything assayed on X or Y chromosome may be taken in consideration and normalized by multiplying the value by two.

2) Cell lines might have a mixed polyploidy and, in this case, the %linkage value may be used as a determinant for targeted integration.

This %linkage calculation can be adopted to check vector integrity of any kind. The two assays will be designed within the vector sequence at the two extremities.

The percent of episomal, off targets driven and random integrated donor DNA (%Episomal/OT) is calculated from the single positive droplets normalized by the genomic integrity and the amount of the control assay.

$$\lambda episomal = \ln(D_{tot}) - \ln(D_{tot} - nD_{DN})$$

$$\lambda control = \ln(D_{tot}) - \ln(D_{notcontrol})$$

$$\%Episomal/OT = \lambda episomal / \lambda control * 100$$

3rd assay. Large deletions and chromosomal aberrations

Evaluation of large deletions per side:

From the flanking assays:

$$Large\ deletions(5')\% = (Norm2_{5'} - 1) * 100$$

$$Large\ deletions(3')\% = (Norm2_{3'} - 1) * 100$$

$$Avg\ Large\ deletions\% = (Large\ deletions_{5'}\% - Large\ deletions_{3'}\%)/2$$

This evaluation will discern the large deletions from the two sides in relation to the cleavage site.

Evaluation of large deletions overall:

From the flanking and the INDELS assays:

$$LargeDelAbs = (Norm2_{distal} - NormLinkageLoss)$$

Those mutations will remove biases derived from the targeted integration or other mutations.

**Claims**

1. A method for quantifying mutation events associated with a targeted genetic modification arranged to modify a target site of a nucleic acid, such as DNA or RNA,

the method comprising carrying out a mutation event determination on a targeted nucleic acid in a population of modified nucleic acids that have been treated with the targeted genetic modification, and a reference control analysis on a non-targeted nucleic acid,
wherein the reference control analysis comprises the use of digital droplet PCR (dPCR) with first and second primer pairs designed to amplify respective first and second regions of the non-targeted nucleic acid and in an unmodified nucleic acid as a control,
wherein the dPCR further comprises a first labelled probe arranged to hybridise with and assay the level of the amplified first region of DNA and a second labelled probe arranged to hybridise with and assay the level of the amplified second region of DNA, wherein the labels of the first and second labelled probes are different to each other,
wherein the relative quantity of dPCR droplets having combined first and second labelled probe detections relative to the quantity of dPCR droplets having first-only or second-only labelled probe detections is determined to quantify the level of genetic integrity of the non-targeted nucleic acid; and
wherein the mutation event determination on a modified nucleic acid that has been treated with the targeted genetic modification comprises one or more analysis strategies selected from:

1) a flanking analysis to determine one or more mutation events including open ends, translocations, and deletions, wherein the flanking analysis is conducted on the modified nucleic acid population and an unmodified nucleic acid population as a control,

the flanking dPCR analysis comprising the use of dPCR with a third primer pair to amplify a 5' region of DNA that is 5' to the target cleavage/editing site in the targeted nucleic acid and a fourth primer pair to amplify a 3' region of DNA that is 3' to the target cleavage/editing site,
wherein the dPCR further comprises a third labelled probe arranged to hybridise with the amplified 5' region of the targeted nucleic acid and a fourth labelled probe arranged to hybridise with the amplified 3' region of the targeted nucleic acid, wherein the labels of the third and fourth labelled probes are different to each other,
wherein the relative level of amplified 5' and 3' regions of the targeted nucleic acid is determined by measuring the quantity of dPCR droplets having combined third and fourth (5' and 3') labelled probe detections relative to the quantity of dPCR droplets having third(5')-only or fourth(3')-only labelled probe detections to quantify the level of mutation events, and optionally, wherein the level of mutation events in the targeted nucleic acid is normalised against the level of genetic integrity of the non-targeted nucleic acid;

2) an on-target analysis to determine mutation events of aberrant insertions and/or deletions, wherein the on-target analysis is conducted on the modified nucleic acid population and an unmodified nucleic acid population as a control,

the on-target analysis comprising the use of dPCR with a fifth primer pair to amplify a region of DNA that includes the target cleavage/editing site in the targeted nucleic acid,
wherein the dPCR further comprises a fifth labelled probe arranged to hybridise with the target cleavage/editing site in the amplified DNA that has been modified by the targeted genetic modification and a sixth labelled probe arranged to hybridise with the amplified DNA at a site that is not the target cleavage/editing site, wherein the labels of the fifth and sixth labelled probes are different to each other, wherein the level of mutation events associated with aberrant deletions and/or insertions at the target cleavage/editing site is determined by measuring the quantity of dPCR droplets having combined fifth and sixth (on-target and off-target) labelled probe detections indicating the presence of the expected genetic modification relative to the quantity of dPCR droplets having fifth(on-target)-only or sixth(off-target)-only labelled probe detections;

3) a knock-in and off-target integration (KI-OT) analysis to determine the level of events associated with integration of a donor DNA into the targeted nucleic acid and/or donor DNA present as separate DNA, wherein the KI-OT analysis is conducted on the modified nucleic acid population and an unmodified nucleic acid population as a control,

the KI-OT analysis comprising the use of dPCR with a primer pair to amplify a region of the donor DNA and a primer pair to amplify a region of the genomic DNA of the targeted nucleic acid, wherein the dPCR further comprises a labelled probe arranged to hybridise with the amplified region of the donor DNA and a labelled probe arranged to hybridise with the targeted nucleic acid, wherein the labels of the two labelled probes are different to each other, wherein the level of integration of the donor DNA into the targeted nucleic acid and/or donor DNA present as separate DNA is determined by determining the quantity of dPCR droplets having combined donor and targeted nucleic acid labelled probe detections, indicating linkage/integration, relative to the quantity of dPCR droplets having donor-only or targeted nucleic acid-only labelled probe detections.

2. The method according to claim 1, wherein the population of modified nucleic acids is nucleic acid in a cell or virus population.

3. The method according to claim 1 or 2, wherein the targeted and/or non-targeted nucleic acid is selected from genomic DNA or RNA, vector DNA, or chromosomal DNA.

4. The method according to any preceding claim, wherein the targeted and/or non-targeted nucleic acid is microbial nucleic acid, optionally viral DNA or RNA.

5. The method according to claim 1, wherein the method comprises the analysis strategies 1 and 2; or wherein the method comprises the analysis strategies 1, 2 and 3.

6. The method according to claim 1, wherein the population of modified nucleic acids is nucleic acid in a modified cell population and wherein the targeted and/or non-targeted nucleic acid is chromosomal DNA; the method may further comprise:

4) a loss of heterozygosity (LOH) analysis to determine the level of mutation events associated with aberrant chromosomal LOH in the targeted chromosome, wherein the LOH analysis is conducted on the modified cell population and an unmodified cell population as a control, the LOH analysis comprising the use of dPCR with a sixth primer pair and a seventh primer pair to amplify respective 5' and 3' sub-telomeric regions of DNA at the extremities of the targeted chromosome, wherein the dPCR further comprises a seventh labelled probe arranged to hybridise with and assay the level of amplified DNA of the 5' sub-telomeric region and an eighth labelled probe arranged to hybridise with and assay the level of amplified DNA of the 3' sub-telomeric region, wherein the labels of the seventh and eighth labelled probes are different to each other, wherein the level of mutation events associated with LOH is determined by the copy number variation of either of the two LOH amplicons (5' and 3' sub-telomeric regions) in relation to the copy number of either of the amplicons as determined in the reference control analysis and the unmodified cell population control.

7. The method according to any preceding claim, wherein the targeted genetic modification comprises the use of a targeted nuclease; or wherein the targeted genetic modification comprises or consists of the use of a RNA-guided endonuclease (RGEN), a zinc finger nuclease (ZFN), a transcription activator-like effector nuclease (TALEN), a base-editor, a prime-editor or a targeted transposon.

8. The method according to any preceding claim, wherein the target cleavage/editing site is in a gene or regulatory sequence; or wherein the target cleavage/editing site is in gene region Xp11 or Xq22.

9. The method according to any preceding claim, wherein the fifth labelled probe is arranged to hybridise with a sequence spanning the cleavage/edited site, wherein the sequence is the unmodified sequence before the genetic modification or a modified sequence following the genetic modification.

10. The method according to any preceding claim, wherein one or more of the probes comprises a minor groove binding domain.

11. The method according to any preceding claim, wherein the method further comprises:

E) a knock-in analysis to determine the level of events associated with integration of a donor DNA into targeted genomic DNA of the targeted nucleic acid,
the knock-in analysis comprising the use of dPCR with a primer pair to amplify a region of DNA comprising genomic and donor DNA, wherein the amplified region spans the join between the genomic DNA and the donor DNA,
wherein the dPCR further comprises a labelled probe arranged to hybridise with the amplified region of genomic and donor DNA,
wherein the level of the amplified region of the genomic DNA and donor DNA in the targeted nucleic acid of the population of modified nucleic acids indicates the level of integration of the donor DNA into the genomic DNA.

12. The method according to any preceding claim, wherein two or more, or all, of the analysis strategies on the treated nucleic acid are carried out in separate dPCR reactions; or
wherein two or more analysis strategies are provided in the same dPCR reaction.

13. The method according to any preceding claim, wherein the label of the labelled probes is a fluorescent label; and/or wherein the Tm of the labelled probes is about 5-15 °C higher than the primers.

14. The method according to claim 6, wherein the cells are associated with a mutation or infection causing a disease or condition; or

wherein the DNA of the unmodified and modified population of nucleic acids is extracted from the cells by glass-bead precipitation, or by using a salting-out genomic DNA extraction method; or
wherein extracted DNA from the cell population is digested into smaller fragments by restriction enzyme digestion prior to distribution in the dPCR droplets.

15. Use of the method according to any preceding claim for screening of potential targeted genetic modification agents for therapeutic use.

**Patentansprüche**

1. Verfahren zum Quantifizieren von Mutationsereignissen, die einer gezielten genetischen Modifikation verknüpft sind, die darauf ausgelegt ist, eine Zielstelle einer Nukleinsäure wie DNA oder RNA zu modifizieren,

wobei das Verfahren das Durchführen einer Mutationsereignisbestimmung an einer gezielten Nukleinsäure innerhalb einer Population modifizierter Nukleinsäuren, die mit der gezielten genetischen Modifikation behandelt wurden, sowie eine Referenzkontrollanalyse an einer nicht gezielten Nukleinsäure umfasst,
wobei die Referenzkontrollanalyse die Verwendung von Digital-Droplet-PCR (dPCR) mit einem ersten und einem zweiten Primerpaar umfasst, die jeweils zur Amplifikation einer ersten bzw. zweiten Region der nicht abgezielten Nukleinsäure ausgelegt sind, sowie an einer unmodifizierten Nukleinsäure als Kontrolle,
wobei die dPCR ferner eine erste markierte Sonde, die zur Hybridisierung mit der amplifizierten ersten Region von DNA und zur Untersuchung deren Niveaus ausgelegt ist, sowie eine zweite markierte Sonde umfasst, die zur Hybridisierung mit der amplifizierten zweiten Region von DNA und zur Untersuchung deren Niveaus ausgelegt ist, wobei sich die Markierungen der ersten und zweiten markierten Sonde voneinander unterscheiden,
wobei die relative Menge an dPCR-Tropfen mit kombinierten Nachweisen der ersten und der zweiten markierten Sonde im Vergleich zur Menge an dPCR-Tropfen mit Nachweisen nur der ersten oder nur der zweiten markierten Sonde bestimmt wird, um das Niveau der genetischen Integrität der nicht abgezielten Nukleinsäure zu quantifizieren; und
wobei die Mutationsereignisbestimmung an einer modifizierten Nukleinsäure, die mit der gezielten genetischen Modifikation behandelt wurde, eine oder mehrere Analysestrategien umfasst, ausgewählt aus:

1) einer Flanking-Analyse zur Bestimmung eines oder mehrerer Mutationsereignisse, einschließlich offener Enden, Translokationen und Deletionen, wobei die Flanking-Analyse an der modifizierten Nukleinsäurepopulation und einer unmodifizierten Nukleinsäurepopulation als Kontrolle durchgeführt wird, wobei die Flanking-dPCR-Analyse die Verwendung von dPCR mit einem dritten Primerpaar zur Amplifikation einer 5'-Region von DNA umfasst, die 5' der Ziel-Spaltungs-/Editierstelle in der gezielten Nukleinsäure liegt, und einem vierten Primerpaar zur Amplifikation einer 3'-Region von DNA umfasst, die 3' der Ziel-Spaltungs-/Editierstelle liegt,

> wobei die dPCR ferner eine dritte markierte Sonde umfasst, die zur Hybridisierung mit der amplifizierten 5'-Region der gezielten Nukleinsäure ausgelegt ist, und eine vierte markierte Sonde, die zur Hybridisierung mit der amplifizierten 3'-Region der gezielten Nukleinsäure ausgelegt ist, wobei sich die Markierungen der dritten und vierten markierten Sonde voneinander unterscheiden,
> wobei das relative Niveau der amplifizierten 5'- und 3'-Regionen der gezielten Nukleinsäure durch Messen der Menge an dPCR-Tröpfchen mit kombiniertem Nachweisen der dritten und vierten (5'- und 3'-) markierten Sonden im Verhältnis zur Menge an dPCR-Tröpfchen mit Nachweis nur von dritter (5') oder vierter (3') markierten Sonde bestimmt wird, um das Niveau an Mutationsereignissen zu quantifizieren, und optional wobei das Niveau an Mutationsereignissen in der gezielten Nukleinsäure gegen das Niveau der genetischen Integrität der nicht gezielten Nukleinsäure normalisiert wird;

2) einer On-Target-Analyse zur Bestimmung von Mutationsereignissen abweichender Insertionen und/oder Deletionen, wobei die On-Target-Analyse an der modifizierten Nukleinsäurepopulation und einer unmodifizierten Nukleinsäurepopulation als Kontrolle durchgeführt wird,

> wobei die On-Target-Analyse die Verwendung von dPCR mit einem fünften Primerpaar zur Amplifikation einer Region von DNA umfasst, die die Ziel-Spaltungs-/Editierstelle in der gezielten Nukleinsäure aufweist,
> wobei die dPCR ferner eine fünfte markierte Sonde umfasst, die zur Hybridisierung mit der Ziel-Spaltungs-/Editierstelle in der amplifizierten DNA ausgelegt ist, die durch die gezielte genetische Modifikation modifiziert wurde, und eine sechste markierte Sonde umfasst, die zur Hybridisierung mit der amplifizierten DNA an einer Stelle ausgelegt ist, die nicht die Ziel-Spaltungs-/Editierstelle ist, wobei sich die Markierungen der fünften und sechsten markierten Sonden voneinander unterscheiden,
> wobei das Niveau an Mutationsereignissen, die mit abweichenden Deletionen und/oder Insertionen an der Ziel-Spaltungs-/Editierstelle verknüpft sind, durch Messen der Menge an dPCR-Tröpfchen mit kombinierten Nachweisen der fünften und sechsten (On-Target- und Off-Target-) markierten Sonden, was auf das Vorhandensein der erwarteten genetischen Modifikation hinweist, im Verhältnis zur Menge an dPCR-Tröpfchen mit Nachweisen von nur fünfter (On-Target-) oder nur sechster (Off-Target-) markierter Sonde bestimmt wird;

3) einer Knock-in- und Off-Target-Integrationsanalyse (KI-OT-Analyse) zur Bestimmung des Niveaus von Ereignissen, die mit der Integration einer Donor-DNA in die gezielte Nukleinsäure und/oder der als separate DNA vorhandenen Donor-DNA verknüpft sind, wobei die KI-OT-Analyse an der modifizierten Nukleinsäurepopulation und einer unmodifizierten Nukleinsäurepopulation als Kontrolle durchgeführt wird, wobei die KI-OT-Analyse die Verwendung von dPCR mit einem Primerpaar zur Amplifikation einer Region der Donor-DNA und einem Primerpaar zur Amplifikation einer Region der genomischen DNA der gezielten Nukleinsäure umfasst,

> wobei die dPCR ferner eine markierte Sonde umfasst, die zur Hybridisierung mit der amplifizierten Region der Donor-DNA ausgelegt ist, und eine markierte Sonde umfasst, die zur Hybridisierung mit der gezielten Nukleinsäure ausgelegt ist, wobei sich die Markierungen der zwei markierten Sonden voneinander unterscheiden,
> wobei das Niveau der Integration der Donor-DNA in die gezielte Nukleinsäure und/oder der als separate DNA vorhandenen Donor-DNA durch Bestimmen der Menge an dPCR-Tröpfchen mit kombinierten Nachweisen von mit Donor- und gezielter Nukleinsäure markierten Sonden, was auf eine Verknüpfung/Integration hinweist, im Verhältnis zur Menge an dPCR-Tröpfchen mit Nachweis von nur mit Donor- oder nur gezielter Nukleinsäure markierter Sonde bestimmt wird.

2. Verfahren nach Anspruch 1, wobei die Population modifizierter Nukleinsäuren eine Nukleinsäure in einer Zelloder Viruspopulation ist.

3. Verfahren nach Anspruch 1 oder 2, wobei die gezielte und/oder nicht gezielte Nukleinsäure aus genomischer DNA oder RNA, Vektor-DNA oder chromosomaler DNA ausgewählt ist.

4. Verfahren nach einem vorhergehenden Anspruch, wobei die gezielte und/oder nicht gezielte Nukleinsäure mikrobielle Nukleinsäure ist, optional virale DNA oder RNA.

5. Verfahren nach Anspruch 1, wobei das Verfahren die Analysestrategien 1 und 2 umfasst; oder
wobei das Verfahren die Analysestrategien 1, 2 und 3 umfasst.

6. Verfahren nach Anspruch 1, wobei die Population modifizierter Nukleinsäuren Nukleinsäure in einer modifizierten Zellpopulation ist und wobei die gezielte und/oder nicht gezielte Nukleinsäure chromosomale DNA ist; wobei das Verfahren ferner umfassen kann:

4) eine Analyse des Verlusts der Heterozygotie (LOH) zur Bestimmung des Niveaus von Mutationsereignissen, die mit einem abweichenden chromosomalen LOH im Zielchromosom verknüpft sind, wobei die LOH-Analyse an der modifizierten Zellpopulation und einer unmodifizierten Zellpopulation als Kontrolle durchgeführt wird,
die LOH-Analyse die Verwendung von dPCR mit einem sechsten Primerpaar und einem siebten Primerpaar zur Amplifikation jeweiliger 5'- und 3'-subtelomerischer Regionen von DNA an den Enden des Zielchromosoms umfasst,
wobei die dPCR ferner eine siebte markierte Sonde umfasst, die zur Hybridisierung mit der amplifizierten DNA der 5'-subtelomerischen Region und der Untersuchung deren Niveaus ausgelegt ist, und eine achte markierte Sonde umfasst, die zur Hybridisierung mit der amplifizierten DNA der 3'-subtelomerischen Region und zur Analyse deren Niveaus ausgelegt ist, wobei sich die Markierungen der siebten und achten markierten Sonden voneinander unterscheiden,
wobei das Niveau der mit LOH verknüpften Mutationsereignisse durch die Kopienzahlvariation eines der beiden LOH-Amplicons (5'- und 3'-subtelomerische Regionen) im Verhältnis zur Kopienzahl eines der Amplicons bestimmt wird, wie in der Referenzkontrollanalyse und der unmodifizierten Zellpopulation als Kontrolle bestimmt.

7. Verfahren nach einem vorhergehenden Anspruch, wobei die gezielte genetische Modifikation die Verwendung einer gezielten Nuklease umfasst; oder
wobei die gezielte genetische Modifikation die Verwendung einer RNA-geleiteten Endonuklease (RGEN), einer Zinkfingernuklease (ZFN), einer Transkriptionsaktivator ähnlichen Effektornuklease (TALEN), eines Basen-Editors, eines Prime-Editors oder eines gezielten Transposons umfasst oder daraus besteht.

8. Verfahren nach einem vorhergehenden Anspruch, wobei sich die Ziel-Spaltungs-/Editierstelle in einem Gen oder einer regulatorischen Sequenz befindet; oder
wobei sich die Ziel-Spaltungs-/Editierstelle in der Genregion Xp11 oder Xq22 befindet.

9. Verfahren nach einem vorhergehenden Anspruch, wobei die fünfte markierte Sonde so ausgelegt ist, dass sie mit einer Sequenz hybridisiert, die sich über die Spaltungs-/editierte Stelle erstreckt, wobei die Sequenz die unmodifizierte Sequenz vor der genetischen Modifikation oder eine modifizierte Sequenz nach der genetischen Modifikation ist.

10. Verfahren nach einem vorhergehenden Anspruch, wobei eine oder mehrere der Sonden eine Kleine-Furche-Bindungsdomäne enthalten.

11. Verfahren nach einem vorhergehenden Anspruch, wobei das Verfahren ferner umfasst:

E) eine Knock-in-Analyse zur Bestimmung des Niveaus von Ereignissen, die mit der Integration einer Donor-DNA in die gezielte genomische DNA der gezielten Nukleinsäure verknüpft sind,
wobei die Knock-in-Analyse die Verwendung von dPCR mit einem Primerpaar zur Amplifikation einer DNA-Region umfasst, die genomische und Donor-DNA enthält, wobei die amplifizierte Region die Verbindungsstelle zwischen der genomischen DNA und der Donor-DNA überspannt,
wobei die dPCR ferner eine markierte Sonde umfasst, die zur Hybridisierung mit der amplifizierten Region von genomischer und Donor-DNA ausgelegt ist,
wobei das Niveau der amplifizierten Region der genomischen DNA und der Donor-DNA in der gezielten Nukleinsäure der Population modifizierter Nukleinsäuren das Niveau der Integration der Donor-DNA in die genomische DNA anzeigt.

**12.** Verfahren nach einem vorhergehenden Anspruch, wobei zwei oder mehr oder alle Analysestrategien an der behandelten Nukleinsäure in getrennten dPCR-Reaktionen durchgeführt werden; oder
wobei zwei oder mehr Analysestrategien in derselben dPCR-Reaktion bereitgestellt werden.

**13.** Verfahren nach einem vorhergehenden Anspruch, wobei die Markierung der markierten Sonden eine Fluoreszenzmarkierung ist;
und/oder
wobei die Tm der markierten Sonden etwa 5 bis 15 °C höher als jene der Primer ist.

**14.** Verfahren nach Anspruch 6, wobei die Zellen mit einer Mutation oder Infektion verknüpft sind, die eine Krankheit oder einen Zustand verursacht; oder

wobei die DNA der unmodifizierten und modifizierten Population von Nukleinsäuren aus den Zellen durch Glasperlenfällung oder unter Verwendung eines Genom-DNA-Extraktionsverfahrens mittels Aussalzens extrahiert wird; oder
wobei die extrahierte DNA aus der Zellpopulation vor der Verteilung in den dPCR-Tröpfchen durch Restriktionsenzymverdau in kleinere Fragmente verdaut wird.

**15.** Verwendung des Verfahrens nach einem vorhergehenden Anspruch zum Screening potenzieller Mittel zur genetischen Modifikation für therapeutische Zwecke.

**Revendications**

**1.** Procédé de quantification d'événements de mutation associés à une modification génétique ciblée agencée pour modifier un site cible d'un acide nucléique, tel que l'ADN ou l'ARN,

le procédé comprenant la mise en œuvre d'un événement de mutation sur un acide nucléique ciblé dans une population d'acides nucléiques modifiés ayant été traités par la modification génétique ciblée, et une analyse de contrôle de référence sur un acide nucléique non ciblé,
dans lequel l'analyse de contrôle de référence comprend l'utilisation d'une PCR en gouttelette numérique (dPCR) avec des première et seconde paires d'amorces conçues pour amplifier les première et seconde régions respectives de l'acide nucléique non ciblé et dans un acide nucléique non modifié comme témoin,
dans lequel la dPCR comprend en outre une première sonde marquée agencée pour s'hybrider avec la première région d'ADN amplifiée et doser le niveau de la première région d'ADN amplifiée et une seconde sonde marquée agencée pour s'hybrider avec la seconde région d'ADN amplifiée et doser le niveau de la seconde région d'ADN amplifiée, dans lequel les marqueurs des première et seconde sondes marquées sont différents l'un de l'autre,
dans lequel la quantité relative de gouttelettes dPCR ayant des détections combinées de première et seconde sondes marquées par rapport à la quantité de gouttelettes dPCR présentant des détections des première et seconde sondes marquées uniquement est déterminée pour quantifier le niveau d'intégrité génétique de l'acide nucléique non ciblé ; et
dans lequel la détermination de l'événement de mutation sur un acide nucléique modifié ayant été traité par la modification génétique ciblée comprend une ou plusieurs stratégies d'analyse sélectionnées parmi :

1) une analyse flanquante pour déterminer un ou plusieurs événements de mutation comprenant des extrémités ouvertes, des translocations et des délétions, dans lequel l'analyse flanquante est effectuée sur la population d'acides nucléiques modifiée et une population d'acides nucléiques non modifiée comme témoin,

l'analyse dPCR flanquante comprenant l'utilisation d' une dPCR avec une troisième paire d'amorces pour amplifier une région 5' de l'ADN qui est à 5' du site de clivage/d'édition cible dans l'acide nucléique ciblé et une quatrième paire d'amorces pour amplifier une région 3' de l'ADN qui est à 3' du site de clivage/d'édition cible,
dans lequel la dPCR comprend en outre une troisième sonde marquée agencée pour s'hybrider avec la région 5' amplifiée de l'acide nucléique ciblé et une quatrième sonde marquée agencée pour s'hybrider avec la région 3' amplifiée de l'acide nucléique ciblé, dans lequel les marqueurs des troisième et quatrième sondes marquées sont différents l'un de l'autre,
dans lequel le niveau relatif des régions 5' et 3' amplifiées de l'acide nucléique ciblé l'acide nucléique est déterminé en mesurant la quantité de gouttelettes dPCR ayant des détections combinées de troisième et quatrième sondes marquées (5' et 3') par rapport à la quantité de gouttelettes dPCR ayant des détections de troisième sonde marquée (5') uniquement ou de quatrième sonde marquée (3') uniquement pour quantifier le niveau d'événements de mutation, et éventuellement, dans lequel le niveau d'événements de mutation dans l'acide nucléique ciblé est normalisé par rapport au niveau d'intégrité génétique de l'acide nucléique non ciblé ;

2) une analyse ciblée pour déterminer les événements de mutation d'insertions et/ou de délétions aberrantes, dans lequel l'analyse ciblée est réalisée sur la population d'acides nucléiques modifiés et une population d'acides nucléiques non modifiés comme témoin,

l'analyse ciblée comprenant l'utilisation de la dPCR avec une cinquième paire d'amorces pour amplifier une région d'ADN qui comprend le site de clivage/d'édition cible dans l'acide nucléique ciblé,
dans lequel la dPCR comprend en outre une cinquième sonde marquée agencée pour s'hybrider avec le site de clivage/d'édition cible dans l'ADN amplifié qui a été modifié par la modification génétique ciblée et une sixième sonde marquée agencée pour s'hybrider avec l'ADN amplifié à un site qui n'est pas le site de clivage/d'édition cible, dans lequel les marqueurs des cinquième et sixième sondes marquées sont différents l'un de l'autre,
dans lequel le niveau d'événements de mutation associés aux délétions et/ou aux insertions aberrantes au site de clivage/d'édition cible est déterminé en mesurant la quantité de gouttelettes dPCR ayant des détections combinées de cinquième et sixième sondes marquées (sur cible et hors cible) indiquant la présence de la modification génétique attendue par rapport à la quantité de gouttelettes dPCR ayant uniquement des détections de cinquième sonde marquée (sur cible) ou sixième sonde marquée (hors cible) ;

3) une analyse d'intégration Knock-in hors cible (KI-OT) pour déterminer le niveau d'événements associés à l'intégration d'un ADN donneur dans l'acide nucléique ciblé et/ou un ADN donneur présent sous forme d'ADN séparé, dans lequel l'analyse KI-OT est réalisée sur la population d'acides nucléiques modifiés et une population d'acides nucléiques non modifiés comme témoin,

l'analyse KI-OT comprenant l'utilisation d'une dPCR avec une paire d'amorces pour amplifier une région de l'ADN donneur et une paire d'amorces pour amplifier une région de l'ADN génomique de l'acide nucléique ciblé,
dans lequel la dPCR comprend en outre une sonde marquée agencée pour s'hybrider avec la région amplifiée de l'ADN donneur et une sonde marquée agencée pour s'hybrider avec l'acide nucléique ciblé, dans lequel les marqueurs des deux sondes marquées sont différents l'un de l'autre,
dans lequel le niveau d'intégration de l'ADN donneur dans l'acide nucléique ciblé et/ou un ADN donneur présent sous forme d'ADN séparé est déterminé en déterminant la quantité de gouttelettes dPCR ayant des détections combinées de sondes marquées d'acides nucléiques donneur et ciblé, indiquant une liaison/intégration, par rapport à la quantité de gouttelettes dPCR ayant des détections de sondes marquées uniquement par le donneur ou uniquement par l'acide nucléique ciblé.

2. Procédé selon la revendication 1, dans lequel la population d'acides nucléiques modifiés est un acide nucléique dans une population de cellules ou de virus.

3. Procédé selon la revendication 1 ou 2, dans lequel l'acide nucléique ciblé et/ou non ciblé est choisi parmi l'ADN ou l'ARN génomique, l'ADN vecteur ou l'ADN chromosomique.

4. Procédé selon l'une quelconque des revendications précédentes, dans lequel l'acide nucléique ciblé et/ou non ciblé est un acide nucléique microbien, éventuellement un ADN ou un ARN viral.

5. Procédé selon la revendication 1, dans lequel le procédé comprend les stratégies d'analyse 1 et 2 ; ou dans lequel le procédé comprend les stratégies d'analyse 1, 2 et 3.

6. Procédé selon la revendication 1, dans lequel la population d'acides nucléiques modifiés est un acide nucléique dans une population cellulaire modifiée et dans lequel l'acide nucléique ciblé et/ou non ciblé est un ADN chromosomique ; le procédé peut en outre comprendre :

4) une analyse de perte d'hétérozygotie (LOH) pour déterminer le niveau d'événements de mutation associés à une LOH chromosomique aberrante dans le chromosome ciblé, l'analyse LOH étant réalisée sur la population cellulaire modifiée et une population cellulaire non modifiée comme témoin,

l'analyse LOH comprenant l'utilisation d'une dPCR avec une sixième paire d'amorces et une septième paire d'amorces pour amplifier les régions sous-télomériques 5' et 3' respectives de l'ADN aux extrémités du chromosome ciblé,

dans lequel la dPCR comprend en outre une septième sonde marquée agencée pour s'hybrider avec et doser le niveau de l'ADN amplifié de la région sous-télomérique 5' et une huitième sonde marquée agencée pour s'hybrider avec et doser le niveau de l'ADN amplifié de la région sous-télomérique 3', les marqueurs des septième et huitième sondes marquées étant différents l'un de l'autre,

dans lequel le niveau d'événements de mutation associés à la LOH est déterminé par la variation du nombre de copies de l'un ou l'autre des deux amplicons LOH (5' et régions sous-télomériques 3') par rapport au nombre de copies de l'un ou l'autre des amplicons tel que déterminé dans l'analyse de contrôle de référence et le contrôle de la population cellulaire non modifiée.

7. Procédé selon l'une quelconque des revendications précédentes, dans lequel la modification génétique ciblée comprend l'utilisation d'une nucléase ciblée ; ou
dans lequel la modification génétique ciblée comprend ou est constituée par l'utilisation d'une endonucléase guidée par l'ARN (RGEN), d'une nucléase à doigt de zinc (ZFN), d'une nucléase effectrice de type activateur de transcription (TALEN), d'un éditeur de base, d'un éditeur d'amorce ou d'un transposon ciblé.

8. Procédé selon l'une quelconque des revendications précédentes, dans lequel le site de clivage/d'édition cible se trouve dans un gène ou une séquence régulatrice ; ou
dans lequel le site de clivage/d'édition cible se trouve dans la région génétique Xp11 ou Xq22.

9. Procédé selon l'une quelconque des revendications précédentes, dans lequel la cinquième sonde marquée est agencée pour s'hybrider avec une séquence couvrant le site de clivage/édité, dans lequel la séquence est la séquence non modifiée avant la modification génétique ou une séquence modifiée après la modification génétique.

10. Procédé selon l'une quelconque des revendications précédentes, dans lequel une ou plusieurs des sondes comprennent un domaine de liaison de rainure mineure.

11. Procédé selon l'une quelconque des revendications précédentes, dans lequel le procédé comprend en outre :

E) une analyse de knock-in pour déterminer le niveau d'événements associés à l'intégration d'un ADN donneur dans l'ADN génomique ciblé de l'acide nucléique ciblé,

l'analyse de knock-in comprenant l'utilisation d' une dPCR avec une paire d'amorces pour amplifier une région d'ADN comprenant de l'ADN génomique et de l'ADN donneur, dans lequel la région amplifiée couvre la jonction entre l'ADN génomique et l'ADN donneur,

dans lequel la dPCR comprend en outre une sonde marquée agencée pour s'hybrider avec la région amplifiée de l'ADN génomique et de l'ADN donneur,

dans lequel le niveau de la région amplifiée de l'ADN génomique et de l'ADN donneur dans l'acide nucléique ciblé de la population d' acides nucléiques modifiés indique le niveau d'intégration de l'ADN donneur dans l'ADN génomique.

12. Procédé selon l'une quelconque des revendications précédentes, dans lequel deux ou plusieurs, ou la totalité, des stratégies d'analyse sur l'acide nucléique traité sont réalisées dans des réactions dPCR distinctes ; ou
dans lequel deux ou plusieurs stratégies d'analyse sont fournies dans la même réaction dPCR.

13. Procédé selon l'une quelconque des revendications précédentes, dans lequel le marqueur des sondes marquées est un marqueur fluorescent ; et/ou
dans lequel la Tm des sondes marquées est d'environ 5 à 15 °C supérieure à celle des amorces.

14. Procédé selon la revendication 6, dans lequel les cellules sont associées à une mutation ou une infection provoquant une maladie ou un état ; ou

dans lequel l'ADN de la population non modifiée et modifiée d'acides nucléiques est extrait des cellules par précipitation par billes de verre, ou en utilisant un procédé d'extraction d'ADN génomique par relargage ; ou
dans lequel l'ADN extrait de la population cellulaire est digéré en fragments plus petits par digestion par enzyme de restriction avant distribution dans les gouttelettes dPCR.

15. Utilisation du procédé selon l'une quelconque des revendications précédentes pour le criblage d'agents de modification génétique ciblés potentiels pour un usage thérapeutique.

Figure 1

Figure 2

## Figure 3

**A** CRISPR/Cas9 RNP — WAS locus — AAV donor

**B** T7E1 — Indels % — WAS, IL7R, BTK, CCR5

**C** FACS analysis of HR% — GFP+ cells % — WAS, IL7R, BTK

**D** MEGA ddPCR plate for WAS edited sample

**E** Allele % — Episomal donor, Targeted chr. loss, Other Aberrations, Large deletions, Target integration, Indel, wt

EP 4 426 856 B1

Figure 4

Figure 5

Figure 6

Figure 7

**A** 3 hours p.t.

**B** 13 days p.t.

- WT
- Indels
- Large del.
- Other Aberration

RNP amount

EP 4 426 856 B1

Figure 8

EP 4 426 856 B1

Figure 9

52

Figure 10

EP 4 426 856 B1

Figure 11

Figure 12

EP 4 426 856 B1

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021130271 A

**Non-patent literature cited in the description**

- **PENG et al.** *Frontiers in Plant Science*, 2020, vol. 11
- **MILLER et al.** *Nucleic Acids Research*, 1988, vol. 16, 1215
- **REGAN JF et al.** *PlosOne*, 2013